# EUROPEAN PATENT SPECIFICATION

(11) **EP 1 600 499 B1**
(45) Date of publication and mention of the grant of the patent: **19.08.2009**
(21) Application number: 04716788.7
(22) Date of filing: 03.03.2004
(51) Int. Cl.: C12N 1/15, C12N 9/18, C12P 17/04, C12N 15/55

(54) **PROCESS FOR PRODUCING LACTONASE AND UTILIZATION THEREOF**
VERFAHREN ZUR HERSTELLUNG VON LACTONASE UND NUTZUNG DAVON
PROCEDE DE PRODUCTION DE LACTONASE ET UTILISATION DE CELLE-CI

(30) Priority: 03.03.2003 JP 2003055233; 31.10.2003 JP 2003371750
(43) Date of publication of application: 30.11.2005
(73) Proprietor: Daiichi Fine Chemical Co., Ltd., Takaoka-shi, Toyama 933-8511 (JP)
(72) Inventor: SHIMIZU, Sakayu, Kyoto-shi, Kyoto 616-8212 (JP); KATAOKA, Michihiko, Kyoto-shi, Kyoto 606-8322 (JP); SAKAMOTO, Keiji, Takaoka-shi, Toyama 933-8511 (JP)
(74) Representative: von Kreisler, Alek
(86) International application number: PCT/JP2004/002643
(87) International publication number: WO 2004/078951

(56) References cited:
- EP-A1- 0 794 251
- EP-A2- 0 519 229
- WO-A-00/28043
- WO-A2-02/74926
- KATAOKA M ET AL: "OPTICAL RESOLUTION OF RACEMIC PANTOLACTONE WITH A NOVEL FUNGAL ENZYME, LACTONOHYDROLASE" APPLIED MICROBIOLOGY AND BIOTECHNOLOGY, SPRINGER VERLAG, BERLIN, DE, vol. 43, 1995, pages 974-977, XP000938407 ISSN: 0175-7598
- NOTHWEHR S F ET AL: "TARGETING OF PROTEINS INTO THE EUKARYOTIC SECRETORY PATHWAY: SIGNAL PEPTIDE STRUCTURE/FUNCTION RELATIONSHIPS" BIOESSAYS, CAMBRIDGE, GB, vol. 12, no. 10, October 1990 (1990-10), pages 479-484, XP009041589 ISSN: 0265-9247
- HONDA KOHSUKE ET AL: "Expression of the Fusarium oxysporum lactonase gene in Aspergillus oryzae: molecular properties of the recombinant enzyme and its application" APPLIED MICROBIOLOGY AND BIOTECHNOLOGY, vol. 66, no. 5, February 2005 (2005-02), pages 520-526, XP002388620 ISSN: 0175-7598
- KOBAYASHI M. ET AL.: 'Lactone-ring-cleaving enzyme: Genetic analysis, novel RNA editing, and evolutionary implications' PROC. NATL. ACAD. SCI. USA vol. 95, 1998, USA, pages 12787 - 12792, XP002134265
- ALBERTS BRUCE ET AL.: 'Essential saibo seibutsugaku' KAIBUSHIKI KAISHA NANKODO 15 February 1999, pages 467 - 470, XP002904681

### SEQUENCE LISTING

<110> DAIICHI FINE CHEMICAL CO., LTD.
<120> Production of Lactonase and Use Thereof
<130> FC-101PCT
<150> JP 2003-55233
   <151> 2003-03-03
<150> JP 2003-371750
   <151> 2003-10-31
<160> 9
<170> Microsoft Windows XP Notepad
<210> 1
   <211> 29
   <212> DNA
   <213> Artificial Sequence
<220>
   <223> Description of Artificial Sequence: Oligonucleotide to act as a p rimer for PCR
<400> 1
   aactcgagat gccttcttcc atttctgta 29
<210> 2
   <211> 28
   <212> DNA
   <213> Artificial Sequence
<220>
   <223> Description of Artificial Sequence: Oligonucleotide to act as a p rimer for PCR
<400> 2
   aatctagact aatcatagag cttgggac 28
<210> 3
   <211> 30
   <212> DNA
   <213> Artificial Sequence
<220>
   <223> Description of Artificial Sequence:Oligonucleotide to act as a p rimer for PCR
<400> 3
   ttgtcgacgg atccgaaaga tgagaccgct 30
<210> 4
   <211> 35
   <212> DNA
   <213> Artificial Sequence
<220>
   <223> Description of Artificial Sequence: Oligonucleotide to act as a p rimer for PCR
<400> 4
   gaagcttagc catgttgatg ctgatgatgg catcc 35
<210> 5
   <211> 43
   <212> DNA
   <213> Artificial Sequence
<220>
   <223> Description of Artificial Sequence: Oligonucleotide to act as a p rimer for PCR
<400> 5
   catcagcatc aacatggcta agctaagctt ccttctacgg ctc 43
<210> 6
   <211> 36
   <212> DNA
   <213> Artificial Sequence
<220>
   <223> Description of Artificial Sequence: Oligonucleotide to act as a p rimer for PCR
<400> 6
   tggaagaagg catcttgtca gggagtatga aggttg 36
<210> 7
   <211> 34
   <212> DNA
   <213> Artificial Sequence
<220>
   <223> Description of Artificial Sequence: Oligonucleotide to act as a p rimer for PCR
<400> 7
   tactccctga caagatgcct tcttcctttc tgta 34
<210> 8
   <211> 1453
   <212> DNA
   <213> Fusarium oxysporum
<220>
   <221> source
   <222> 1..1453
   <223> /organism="Fusarium oxysporum"
<220>
   <221> mRNA
   <222> join(1.. 153, 203.. 545, 597.. 704, 757.. 966, 1015.. 1191, 1242.. 1453)
   <223>
<220>
   <221> CDS
   <222> join(1.. 153, 203.. 545, 597.. 704, 757.. 966, 1015.. 1191, 1242.. 1453)
   <223>
<220>
   <221> sig_peptide
   <222> (1).. (60)
   <223>
<220>
   <221> mat_peptide
   <222> join(61.. 153, 203.. 545, 597.. 704, 757.. 966, 1015.. 1191, 1242.. 1450)
   <223>
<220>
   <221> exon
   <222> (1).. (153)
   <223>
<220>
   <221> exon
   <222> (203).. (545)
   <223>
<220>
   <221> exon
   <222> (597)..(704)
   <223>
<220>
   <221> exon
   <222> (757).. (966)
   <223>
<220>
   <221> exon
   <222> (1015).. (1191)
   <223>
<220>
   <221> exon
   <222> (1242).. (1453)
   <223>
<220>
   <221> Intron
   <222> (154).. (202)
   <223>
<220>
   <221> Intron
   <222> (546).. (596)
   <223>
<220>
   <221> Intron
   <222> (705)..(756)
   <223>
<220>
   <221> Intron
   <222> (967).. (1014)
   <223>
<220>
   <221> Intron
   <222> (1192).. (1241)
   <223>
<400> 8
   atg cct tct tcc att tct gta ctt gcg ggg gtt ctc gtg cct gtt ttg 48
   Met Pro Ser Ser Ile Ser Val Leu Ala Gly Val Leu Val Pro Val Leu
   -20 -15 -10 -5
   ggc gcg gtg gct gct aag ctt cct tct acg gct cag att att gat cag 96
   Gly Ala Val Ala Ala Lys Leu Pro Ser Thr Ala Gln Ile Ile Asp Gln
   -1 1 5 10
   aag tcg ttc aat gtc ttg aag gat gtg cca cct cct gca gtg gcc aat 144
   Lys Ser Phe Asn Val Leu Lys Asp Val Pro Pro Pro Ala Val Ala Asn
   15 20 25
   gac tct ctg gtatgtcact gcagcctcgt tgctgattgt tactaataat tatgagtag 202
   Asp Ser Leu
   30
   gtg ttc act tgg cct ggt gta act gag gag tct ctt gtt gag aag cct 250
   Val Phe Thr Trp Pro Gly Val Thr Glu Glu Ser Leu Val Glu Lys Pro
   35 40 45
   ttc cat gtc tac gat gaa gag ttt tac gat gta att gga aaa gac ccc 298
   Phe His Val Tyr Asp Glu Glu Phe Tyr Asp Val Ile Gly Lys Asp Pro
   50 55 60
   tct ttg acc ctc atc gca aca tcg gac acc gac cca atc ttc cat gag 346
   Ser Leu Thr Leu Ile Ala Thr Ser Asp Thr Asp Pro Ile Phe His Glu
   65 70 75
   gct gtc gta tgg tat cct cct act gaa gag gtg ttc ttt gtg cag aat 394
   Ala Val Val Trp Tyr Pro Pro Thr Glu Glu Val Phe Phe Val Gln Asn
   80 85 90 95
   gct ggc gct cct gcc gca ggc act ggc ttg aac aag tct tcc atc att 442
   Ala Gly Ala Pro Ala Ala Gly Thr Gly Leu Asn Lys Ser Ser Ile Ile
   100 105 110
   cag aag att tcc ctc aag gag gcc gat gct gtt cgc aag ggc aag cag      490
   Gln Lys Ile Ser Leu Lys Glu Ala Asp Ala Val Arg Lys Gly Lys Gln
               115                 120                 125
   gat gag gtc aag gtc aca gtt gtt gac tcg aac cct cag gtt atc aac      538
   Asp Glu Val Lys Val Thr Val Val Asp Ser Asn Pro Gln Val Ile Asn
           130                 135                 140
   cca aat g gtatgtttaa cgggtgatat gacttaaacg agcctaacct tttgatctca     595
   Pro Asn
       145
   g gt  ggt act tac tac aag ggc aac atc atc ttc gct ggt gag ggc caa     643
     Gly Gly Thr Tyr Tyr Lys Gly Asn Ile Ile Phe Ala Gly Glu Gly Gln
                     150                 155                 160
   ggc gac gat gtt ccc tct gcg ctg tac ctc atg aac cct ctc cct cct      691
   Gly Asp Asp Val Pro Ser Ala Leu Tyr Leu Met Asn Pro Leu Pro Pro
               165                 170                 175
   tac aac acc acc a gtaagtcaca agaatcccta agagcacaat tctttgagct        744
   Tyr Asn Thr Thr
           180
   aaccttaacc ag cc  ctt ctc aac aac tac ttc ggt cgc cag ttc aac tcc    794
                 Thr Leu Leu Asn Asn Tyr Phe Gly Arg Gln Phe Asn Ser
                             185                 190
   ctc aac gac gtc ggt atc aac ccc agg aac ggt gac ctg tac ttc acc      842
   Leu Asn Asp Val Gly Ile Asn Pro Arg Asn Gly Asp Leu Tyr Phe Thr
   195                 200                 205                 210
   gat acc ctc tac gga tat ctc caa gac ttc cgt cct gtt cct ggt ctg      890
   Asp Thr Leu Tyr Gly Tyr Leu Gln Asp Phe Arg Pro Val Pro Gly Leu
                   215                 220                 225
   cga aac cag gtc tat cgt tac aac ttt gac act ggc gct gtc act gtt      938
   Arg Asn Gln Val Tyr Arg Tyr Asn Phe Asp Thr Gly Ala Val Thr Val
               230                 235                 240
   gtc gct gat gac ttt acc ctt ccc aac g gtatgtcttt tattgaatgg          986
   Val Ala Asp Asp Phe Thr Leu Pro Asn
           245                 250
   taaagtacaa gcaactcata ttgatcag gt  att ggc ttt ggc ccc gac ggc      1037
                                   Gly Ile Gly Phe Gly Pro Asp Gly
                                               255
   aag aag gtt tat gtc acc gac act ggc atc gct ctc ggt ttc tac ggt     1085
   Lys Lys Val Tyr Val Thr Asp Thr Gly Ile Ala Leu Gly Phe Tyr Gly
   260                 265                 270                 275
   cgc aac ctc tct tct ccc gct tct gtt tac tct ttc gac gtg aac cag     1133
   Arg Asn Leu Ser Ser Pro Ala Ser Val Tyr Ser Phe Asp Val Asn Gln
                   280                 285                 290
   gac ggt act ctt cag aac cgc aag acc ttt gct tat gtt gcc tca ttc     1181
   Asp Gly Thr Leu Gln Asn Arg Lys Thr Phe Ala Tyr Val Ala Ser Phe
                   295                 300                 305
   atc ccc gat g gtaagtcctg tatgttgtgc ctctgtcgtg ttatatactg        1231
   Ile Pro Asp
           310
   acctctccag gt  gtc cac act gac tcc aag ggt cgt gtt tat gct ggc   1279
              Gly Val His Thr Asp Ser Lys Gly Arg Val Tyr Ala Gly
                              315                 320
   tgc ggt gat ggt gtc cat gtc tgg aac ccc tct ggc aag ctc atc ggc  1327
   Cys Gly Asp Gly Val His Val Trp Asn Pro Ser Gly Lys Leu Ile Gly
       325                 330                 335
   aag atc tac acc gga acg gtt gct gct aac ttc cag ttt gct ggt aag  1375
   Lys Ile Tyr Thr Gly Thr Val Ala Ala Asn Phe Gln Phe Ala Gly Lys
   340                 345                 350                 355
   gga agg atg ata att act gga cag acg aag ttg ttc tat gtc act cta  1423
   Gly Arg Met Ile Ile Thr Gly Gln Thr Lys Leu Phe Tyr Val Thr Leu
                   360                 365                 370
   ggg gct tcg ggt ccc aag ctc tat gat tag                          1453
   Gly Ala Ser Gly Pro Lys Leu Tyr Asp
               375                 380
<210> 9
   <211> 400
   <212> PRT
   <213> Fusarium oxysporum
<400> 9
   Met Pro Ser Ser Ile Ser Val Leu Ala Gly Val Leu Val Pro Val Leu
   -20 -15 -10 -5
   Gly Ala Val Ala Ala Lys Leu Pro Ser Thr Ala Gln Ile Ile Asp Gln
   -1 1 5 10
   Lys Ser Phe Asn Val Leu Lys Asp Val Pro Pro Pro Ala Val Ala Asn
   15 20 25
   Asp Ser Leu Val Phe Thr Trp Pro Gly Val Thr Glu Glu Ser Leu Val
   30 35 40
   Glu Lys Pro Phe His Val Tyr Asp Glu Glu Phe Tyr Asp Val Ile Gly
   45 50 55 60
   Lys Asp Pro Ser Leu Thr Leu Ile Ala Thr Ser Asp Thr Asp Pro Ile
   65 70 75
   Phe His Glu Ala Val Val Trp Tyr Pro Pro Thr Glu Glu Val Phe Phe
   80 85 90
   Val Gln Asn Ala Gly Ala Pro Ala Ala Gly Thr Gly Leu Asn Lys Ser
   95 100 105
   Ser Ile Ile Gln Lys Ile Ser Leu Lys Glu Ala Asp Ala Val Arg Lys
   110 115 120
   Gly Lys Gln Asp Glu Val Lys Val Thr Val Val Asp Ser Asn Pro Gln
   125 130 135 140
   Val Ile Asn Pro Asn Gly Gly Thr Tyr Tyr Lys Gly Asn Ile Ile Phe
   145 150 155
   Ala Gly Glu Gly Gln Gly Asp Asp Val Pro Ser Ala Leu Tyr Leu Met
   160 165 170
   Asn Pro Leu Pro Pro Tyr Asn Thr Thr Thr Leu Leu Asn Asn Tyr Phe
   175 180 185
   Gly Arg Gln Phe Asn Ser Leu Asn Asp Val Gly Ile Asn Pro Arg Asn
   190 195 200
   Gly Asp Leu Tyr Phe Thr Asp Thr Leu Tyr Gly Tyr Leu Gln Asp Phe
   205 210 215 220
   Arg Pro Val Pro Gly Leu Arg Asn Gln Val Tyr Arg Tyr Asn Phe Asp
   225 230 235
   Thr Gly Ala Val Thr Val Val Ala Asp Asp Phe Thr Leu Pro Asn Gly
   240 245 250
   Ile Gly Phe Gly Pro Asp Gly Lys Lys Val Tyr Val Thr Asp Thr Gly
   255 260 265
   Ile Ala Leu Gly Phe Tyr Gly Arg Asn Leu Ser Ser Pro Ala Ser Val
   270 275 280
   Tyr Ser Phe Asp Val Asn Gln Asp Gly Thr Leu Gln Asn Arg Lys Thr
   285 290 295 300
   Phe Ala Tyr Val Ala Ser Phe Ile Pro Asp Gly Val His Thr Asp Ser
   305 310 315
   Lys Gly Arg Val Tyr Ala Gly Cys Gly Asp Gly Val His Val Trp Asn
   320 325 330
   Pro Ser Gly Lys Leu Ile Gly Lys Ile Tyr Thr Gly Thr Val Ala Ala
   335 340 345
   Asn Phe Gln Phe Ala Gly Lys Gly Arg Met Ile Ile Thr Gly Gln Thr
   350 355 360
   Lys Leu Phe Tyr Val Thr Leu Gly Ala Ser Gly Pro Lys Leu Tyr Asp
   365 370 375 380

## Description

### FIELD OF THE INVENTION

The present invention relates to a lactonase-producing transformant and its use in a process for the production of lactonase having D-pantolactone-hydrolyzing enzyme activity and in a process for the production techniques for optically-active γ-lactone and related compounds thereof.

### BACKGROUND OF THE INVENTION

It is known that D-pantolactone (D-pantoyl lactone) is an intermediate for the production of D-pantothenic acid, D-panthenol, and pantethine. These compounds serve as pharmaceutically and physiologically important vitamins which are useful as pharmaceutical drugs, food & feed additives, and raw materials for cosmetics.

In the prior art, D-pantolactone is manufactured via optical resolution of chemically synthesized D,L-pantolactone. However, this method requires expensive resolving agents such as quinine and brucine and is accompanied with drawbacks such as hard recovery of D-pantolactone. For solving these problems an optical resolution technique relying on the enzymatic asymmetric hydrolysis of D,L-pantolactone is provided in Patent Documents 1 and 2.

Briefly, such techniques are D-pantolactone production methods wherein D-form pantolactone included in a D,L-pantolactone mixture is selectively subjected to asymmetric hydrolysis with a lactone-hydrolyzable microorganism selected from those belonging to the genera: Fusarium, Cylindrocarpon, Gibberella, Aspergillus, Penicillium, Rhizopus, Volutella, Gliocladium, Eurotium, Nectria, Schizophyllum, Myrothecium, Neurospora, Acremonium, Tuberculina, Absidia, Sporothrix, Verticillium, and Arthroderma to produce D-pantoic acid which is then separated and converted into D-pantolactone, and preparation methods for D-pantolactone-hydrolyzing enzymes relying on the above microorganisms.

However, a lot of microorganisms disclosed therein not necessarily have sufficient hydrolyzing activity to allow the direct industrial applications.

In order to enhance the enzyme activity owned by the microorganisms to industrially applicable levels, there is a need to conduct time-consuming, complicated and hard investigations on culture conditions, activity-inducing conditions, and others. There are drawbacks that since these microorganisms are fungi, each organism shows filamentous structures with various forms, and it is relatively difficult to prepare immobilized microorganisms which are advantageous in view of industrial production, as compared to bacteria having a uniform style. It is further a drawback that the percent enzyme recovery is quite low for D-pantolactone-hydrolyzing enzymes when the enzymes are purified from microorganisms.

For solving these problems, and further aiming at attaining to significantly improved enzymatic activity via modification of D-pantolactone-hydrolyzing enzyme itself, the gene was disclosed that coded for naturally-occurring D-pantolactone-hydrolyzing enzyme, e.g., Fusarium oxysporum-derived naturally-occurring D-pantolactone-hydrolyzing enzyme or proteins having substantially equivalent activity thereto, and DNA containing a nucleotide sequence coding for said protein was transferred to produce transformed cells (Patent Document 3).

Further, a non-glycosylated lactonohydrolase derived from *Fusarium oxysporum* and a glycosylated lactonohydrolase derived from *Fusarium venenatum* are known from Patent Documents 4 and 5, respectively.

However, it is still desired to develop more efficient methods for allowing direct industrial practices even with the activity exerted by enzymes obtained from said transformed cells.
[Patent Document 1] JP-A-3-65198 (1991)
[Patent Document 2] JP-A-4-144681 (1992)
[Patent Document 3] WO-A-97/10341
[Patent Document 4] WO-A-02/074926
[Patent Document 5] WO-A-00/028043

### SUMMARY OF THE INVENTION

The prior art enzymes produced by transformants obtained with applications of E. coli and the like lack glycosylation, with a smaller molecular mass than naturally-occurring type (wild type) lactonase, and have drawbacks such as a defect in their stability. It is therefore desired to overcome these drawbacks.

An object of the present invention is to construct efficient and excellently productive systems for producing said lactonase enzyme by utilization of the naturally-occurring type (wild type) lactonase gene.

Another object of the present invention is to construct efficient and advantageously productive systems for producing optically-active γ-lactones via use of said enzyme and said enzyme-production system.

The gene analysis of D-pantolactone-hydrolyzing enzyme with D-pantolactone-hydrolyzing enzyme activity (hereinafter, designated as "lactonase"), in particular, lactonase cloned and identified from Fusarium oxysporum, has revealed that the enzyme is encoded by the chromosomal gene consisting of 5 introns composed of 1453 nucleotide pairs and comprises an NH₂-terminal signal peptide composed of 20 amino acid residues, and the mature lactonase protein is encoded by cDNA complementary to mRNA from the transcription of said chromosomal lactonase gene and composed of 380 amino acid residues. And the wild-type lactonase is glycosylated.

In view of the aforementioned problems, the inventor and et al. have conducted an extensive research and investigation in order to develop a system allowing glycosylation in recombinant lactonase enzymes. As a result, the inventors have succeeded in the mass production of glycosylated lactonase enzymes having native form saccharide chains via gene co-transfer of the lactonase gene in combination with a gene sequence coding for a signal peptide into a host cell for gene expression. Based on these findings, the present inventors have accomplished the present invention.

Thus, the present invention provides the following:
(1) A lactonase-producing transformant of the genus *Acremonium* or *Aspergillus* which has genetically acquired a DNA comprised of a lactonase-encoding DNA coding for amino acid residues 1 to 380 of SEQ ID NO:9 and a signal peptide region-encoding DNA coding for amino acid residues -20 to -1 of SEQ ID NO:9.
(2) A process for the production of a recombinant lactonase with D-pantolactone-hydrolyzing enzyme activity, which comprises culturing the lactonase-producing transformant according to the above (1) to produce the recombinant lactonase, and isolating the resultant recombinant lactonase from the culture.
(3) A process for producing an optically-active γ-lactone derivative of the general formula (II): 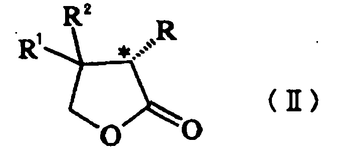 wherein R is hydroxyl or amino, R¹ and R², identical or different each other, are independently hydrogen or lower alkyl, or of the general formula (IV): 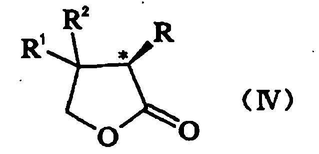 wherein R, R¹ and R², have the same meanings as defined above, or a salt thereof, which comprises contacting a compound of the general formula (I): 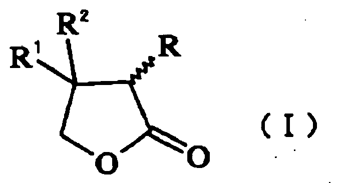 wherein R, R¹ and R², have the same meanings as defined above, or a salt thereof, with a member selected from the group consisting of cell cultures, cells, and immobilized cells of the lactonase-producing transformant according to the above (1), to produce the optically-active γ-lactone derivative (II) or (IV), or a salt thereof.
(4) The process according to the above (3), wherein the optically active γ-lactone of formula (IV) is D-pantolactone, or a salt thereof, and the process allows the production of D-pantolactone derivatives or salts thereof.
(5) The process according to the above (3), wherein the D-pantolactone derivatives are selected from pantothenic acid, calcium pantothenate, panthenol, pantetheine, panthenyl ethyl ether and pantethine.

Preferred embodiments are set forth in the following:

In a first preferred embodiment the lactonase-producing transformant of (1) above has received a gene transfer with DNA coding for not only lactonase having D-pantolactone-hydrolyzing enzyme activity but also a signal peptide region, or a vector containing an insert of said DNA.

In the above lactonase-producing transformant, said vector may carry one or plural filamentous fungal enhancer nucleotide sequences.

In the above lactonase-producing transformant, said vector may have incorporated one or plural filamentous fungal enhancer nucleotide sequences into a promoter region which functions in fungi.

In the above lactonase-producing transformant, said vector may have incorporated one or plural enhancer sequences into a promoter region which functions in fungi, wherein said enhancer sequence is derived from a location on the Aspergillus oryzae α-glucosidase gene (agdA) promoter.

In the above the lactonase-producing transformant, said promoter region may be a member derived from filamentous fungal hydrolyzing enzyme genes or glycolytic pathway enzyme genes.

In the above the lactonase-producing transformant, said promoter region may be an Aspergillus oryzae-derived α-glucosidase gene promoter or a promoter portion comprising part of said α-glucosidase gene promoter.

In the above the lactonase-producing transformant, said vector may be a plasmid for expression in fungi and may comprise a suitable marker gene for selection of transformed filamentous fungal host cells, a terminator, and a DNA region replicable in E. coli.

In the above lactonase-producing transformant, said marker gene may be a filamentous fungal nitrate reductase gene, a filamentous fungal ornithine carbamoyltransferase gene, a filamentous fungal tryptophan synthetase gene, or a filamentous fungal acetamidase gene.

In the above lactonase-producing transformant, said marker gene may be the Aspergillus-derived nitrate reductase gene.

In the above lactonase-producing transformant, said terminator may be an Aspergillus oryzae-derived α-glucosidase gene terminator or a terminator portion comprising part of said α-glucosidase gene terminator.

In the above the lactonase-producing transformant, said vector may comprise a promoter region associated with the expression of Fusarium fungal alkaline protease (Alp).

In the above the lactonase-producing transformant, said signal peptide region may be a signal sequence region associated with the secretion of said Alp or a signal sequence region for said lactonase.

In a second preferred embodiment the lactonase-producing transformant of (1) above has received a gene transfer with DNA comprising (a) a DNA sequence coding for full-length form lactonase with D-pantolactone-hydrolyzing enzyme activity and (b) a DNA sequence coding for an NH₂-terminal signal peptide region of said lactonase.

In the above the lactonase-producing transformant, said host may have received a gene transfer with DNA comprising (a) (i) a cDNA sequence coding for full-length form lactonase or (ii) a chromosomal full-length form lactonase DNA sequence and (b) a DNA sequence coding for an NH₂-terminal signal peptide region of said lactonase.

In a third preferred embodiment, the lactonase-producing transformant of (1) above has received a gene transfer with DNA comprising (a) a DNA sequence coding for full-length form lactonase with D-pantolactone-hydrolyzing enzyme activity and (b) a DNA sequence coding for a Fusarium fungal Alp signal peptide region.

In the above the lactonase-producing transformant said host may have received a gene transfer with DNA comprising (a) (i) a cDNA sequence coding for full-length form lactonase or (ii) a chromosomal full-length form lactonase DNA sequence and (b) a DNA sequence coding for said Alp signal peptide region.

In a fourth preferred embodiment, the lactonase-producing transformant of (1) above has received a gene transfer with DNA comprising DNA with nucleotides 61 to 1453 of Sequence Listing SEQ ID NO: 8 and DNA coding for a signal peptide region thereof.

In the above lactonase-producing transformant said signal peptide region is an amino acid sequence with amino acid residues -20 to -1 of Sequence Listing SEQ ID NO: 9 or a Fusarium fungal Alp signal peptide region.

In a fifth preferred embodiment the process of (3) above is suitable for producing an (S)-γ-lactone derivative or a salt thereof, which comprises
contacting a compound of the general formula (I): 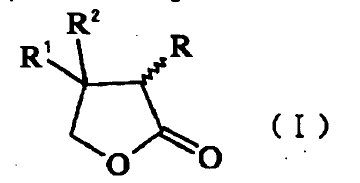 wherein R is hydroxyl or amino, R¹ and R², identical or different each other, are independently hydrogen or lower alkyl, or a salt thereof, with a member selected from the group consisting of cell cultures, cells, processed cell products, and immobilized cells of the lactonase-producing transformant of (1) above, and
then isolating an unreactant compound of the general formula (II): 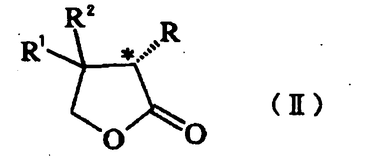 wherein R, R¹ and R², have the same meanings as defined above, or a salt thereof,
from the reaction system.

In a sixth preferred embodiment the process of (3) above is suitable for producing an (R)-γ -lactone derivative or a salt thereof, which comprises
contacting a compound of the general formula (I): 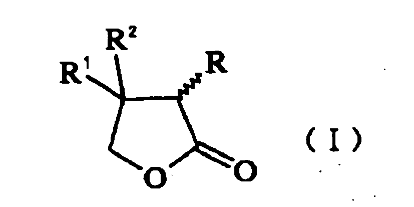 wherein R is hydroxyl or amino, R¹ and R², identical or different each other, are independently hydrogen or lower alkyl, or a salt thereof, with a member selected from the group consisting of cell cultures, cells, processed cell products, and immobilized cells of the lactonase-producing transformant of (1) above to produce a compound of the general formula (III): 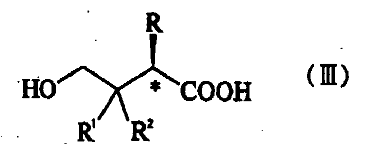 wherein R, R¹ and R², have the same meanings as defined above, or a salt thereof, and
then converting the resultant compound (III) into a compound of the formula (IV): 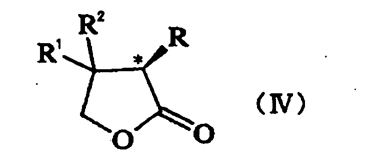 wherein R, R¹ and R², have the same meanings as defined above, or a salt thereof.

In a seventh preferred embodiment recipient host microbial cell for gene transfer of the lactonase-producing transformant of (1) above is a member selected from Aspergillus oryzae and Acremonium chrysogenum.

In an eighth preferred embodiment the process of (3) above is suitable for producing an optically-active γ-lactone derivative of the general formula (II) or (IV), which comprises immobilizing a member selected L from the group consisting of lactonase-producing transformants, cell cultures, cells, processed cell products thereof, and recombinant lactonase enzymes from said transformant, and repeating a reaction with the resultant immobilized product.

In a ninth preferred embodiment the process of (3) above is suitable for producing an optically-active pantolactone, or a salt thereof, which comprises using a compound of the general formula (I) wherein R is hydroxyl, R¹ and R², are both methyl.

In a tenth preferred embodiment the process of (3) above is suitable for producing a member selected from the group consisting of pantothenic acid, or a salt thereof, panthenol, and pantethine, which comprises contacting a compound of the general formula (I), or a salt thereof, with a member selected from the group consisting of cell cultures, cells, processed cell products, and immobilized cells of the lactonase-producing transformant of the above (1) and then treating the resultant D-pantolactone to produce said product member.

In an eleventh preferred embodiment an immobilized microbial cell can be produced by immobilization of a transformed cell (or transformant) of (1) above.

In a twelfth preferred embodiment the process of (3) above is suitable for producing a D-pantolactone derivative, or a salt thereof, which comprises contacting a compound of the formula (I) or a salt thereof with a member selected from the group consisting of cell cultures, cells, processed cell products, and immobilized cells of the lactonase-producing transformant of (1) above, to produce an optically-active compound of the general formula (III) or a salt thereof and then applying the resultant optically-active compound (III) as a starting material to known processes, equivalent treatments thereof, or modifications thereof to produce said D-pantolactone derivative, or a salt thereof, including a compound of the formula (IV), pantothenic acid, calcium pantothenate, panthenol, pantetheine, coenzyme A (CoA), panthenyl ethyl ether, pantethine and others.

In the above process
(a) D-pantolactone is coupled with a Ca salt or ester of β-alanine to produce calcium D-pantothenate,
(b) D-pantolactone is coupled with β-alanine or its ester to produce D-pantothenic acid, and the resultant D-pantothenic acid is then reacted with a calcium compound to produce calcium D-pantothenate,
(c) D-pantolactone is reacted with β-alanine in the presence of a secondary or tertiary amine to produce a reaction mixture, and the resultant mixture is then admixed with calcium oxide to calcium D-pantothenate,
(d) D-pantolactone is coupled with 3-aminopropanol to produce D-panthenol,
(e) D-pantolactone is reacted with γ-aminopropionitrile to produce D-pantothenonitrile, and the resultant nitrile is then reacted with cysteamine to produce 2-(2-D-pantoamidoethyl)-2-thiazoline which is then hydrolyzed to produce D-pantetheine, or
(f) the product D-pantetheine from the aforementioned step (e) is condensed in the presence of hydrogen peroxide to produce pantethine.

### ADVANTAGEOUS PROFILES OF THE INVENTION

The present invention provides efficient and excellently productive techniques for the construction of systems capable of producing said enzyme wherein said system utilizes the native form lactonase gene. The present invention also enables the construction of optically-active γ-lactone production systems with efficient and excellent productivity, utilizing said enzyme and systems for producing said enzyme.
The present invention does not require any expensive resolving agent but allows the simply operable, industrially productive, efficient production of optically-active y -lactone derivatives useful for pharmaceutical drug intermediates and starting materials to produce amino acids and pantothenic acid, with less ecological problems. Simple, convenient and industrial production techniques for optically-active γ-lactone derivatives are provided. Further, the present invention allows the efficient and economically advantageous synthesis of D-pantolactone and derivatives thereof, for example including pantothenic acid, calcium pantothenate, panthenol, pantetheine, coenzyme A (CoA), panthenyl ethyl ether, pantethine and a salt thereof.

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### BRIEF DESCRIPTION OF THE DRAWINGS

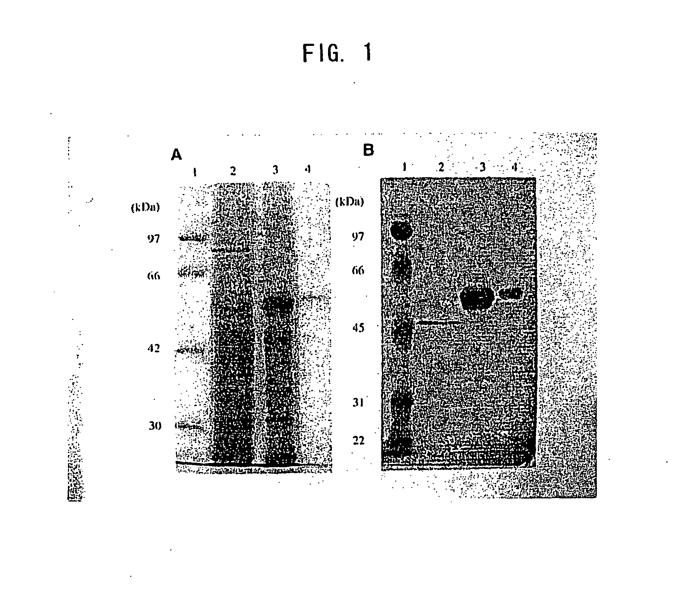

Fig. 1 shows electrophoresis profiles (photos) for expression of the foreign lactonase gene in A. oryzae.
   A: Coomassie brilliant blue-stained gel showing electrophoretically separated proteins. Lane 1, Molecular mass standards (Daiichi Chemicals, Tokyo, Japan): phosphorylase b (97 kDa), bovine serum albumin (66 kDa), aldolase (42 kDa), and carbonic anhydrase (30 kDa). Lane 2, Cell-free extracts (25µ g for each lane) of A. oryzae transformed with pNAN-PC. Lane 3, Cell-free extracts (25µg for each lane) of A. oryzae transformed with pNAN-XG. Lane 4, Purified lactonase from F. oxysporum, 0.5 µg.
   B: Western blot of a similar gel after immunostaining with the antibodies specific for the wild lactonase. Lane 1 was loaded with the following molecular mass standards (prestained SDS-PAGE standards low range, Bio-Rad, Hercules, USA): phosphorylase b (97 kDa), bovine serum albumin (66 kDa), ovalbumin (45 kDa), carbonic anhydrase (31 kDa), trypsin inhibitor (22 kDa).

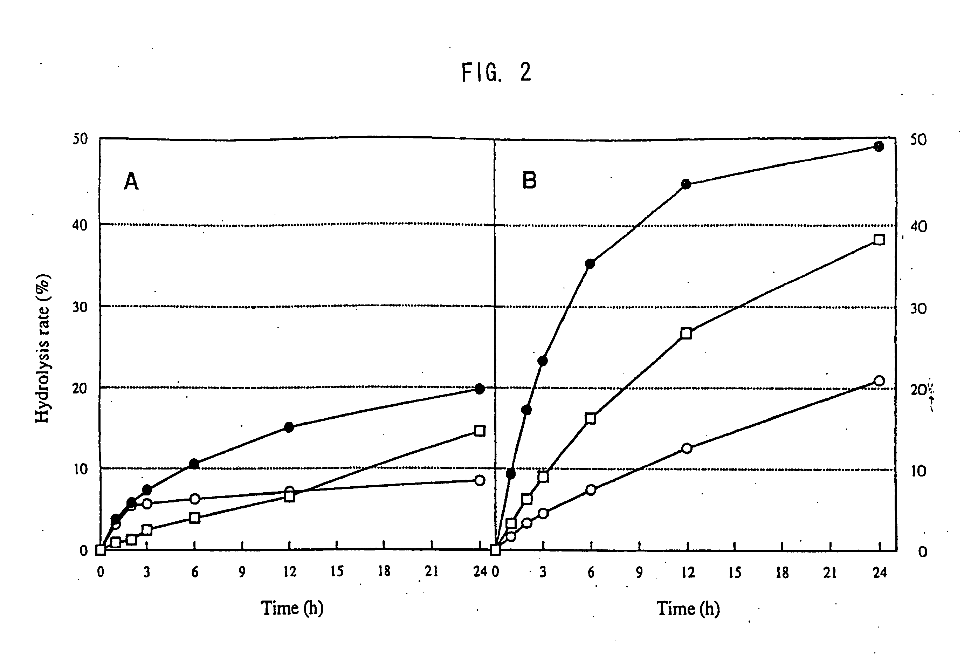

Fig. 2 shows asymmetric hydrolysis results of racemic pantolactone with F. oxysporum and recombinant A. oryzae.
   A: Wet cells. B: Immobilized cells.
   O: A. oryzae transformed with pNAN-PC. ● : A. oryzae transformed with pNAN-XG. □: F. oxysporum.

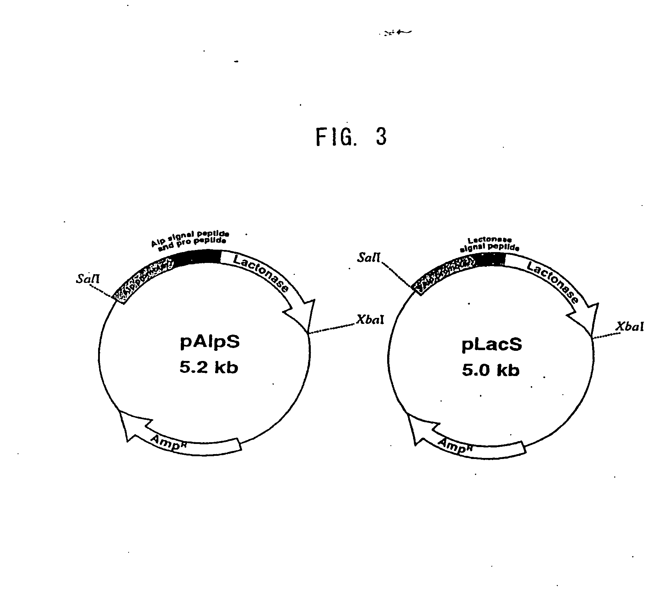

Fig. 3 is construction of the expression plasmids for secretion of the lactonase. The fusion genes were constructed by combined PCRs, digested by SalI and XbaI, and then subcloned into pBluescript II SK+.

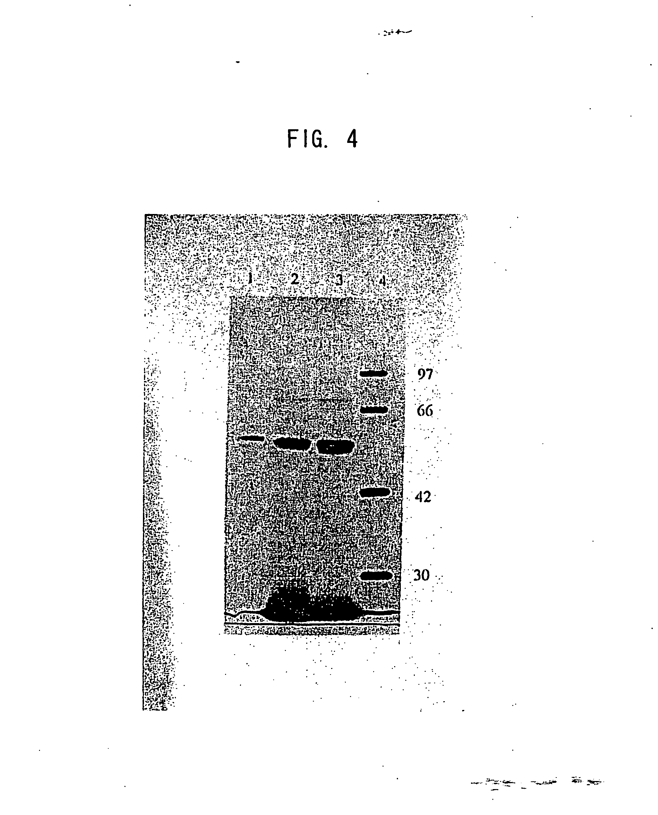

Fig. 4 shows electrophoresis (SDS-PAGE, 10% polyacrylamide gel) profiles (photos) of the lactonase secreted by Ac. chrysogenum transformants. Lane 1: The wild type lactonase (lane 1, 0.5/µg) purified from F. oxysporum. Lane 2: Culture supernatants (containing 3 µg proteins) of Ac. chrysogenum transformed with pAlpS. Lane 3: Culture supernatants (containing 3 µg proteins) of Ac. chrysogenum transformed with pLacS. Lane 4: Molecular mass standards; phosphorylase b (97 kDa), bovine serum albumin (66 kDa), aldolase (42 kDa), and carbonic anhydrase (30 kDa).

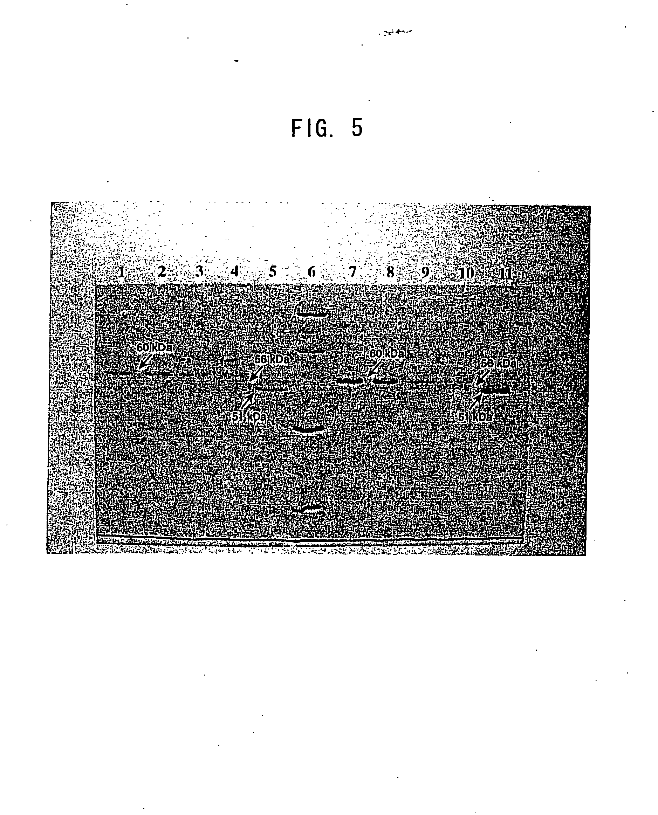

Fig. 5 shows electrophoresis (SDS-PAGE) profiles (photos) of the deglycosylated lactonases. The wild type lactonase (lanes 2 to 5) and the recombinant lactonase (lanes 8 to 11) purified from Ac. chrysogenum transformed with pAlpS were treated with EndoH_{f}, and then subjected to electrophoresis on 10% polyacrylamide gel (0.8 µg for each lane). The deglycosylation was carried out for 1 min (lanes 2 and 8), for 5 min (lanes 3 and 9), for 15 min (lanes 4 and 10), or for 60 min (lanes 5 and 11). Non-deglycosylated wild type and recombinant enzymes were loaded into lanes 1 and 7, respectively. Lane 6: Molecular mass standards (see, Fig.4 for details).

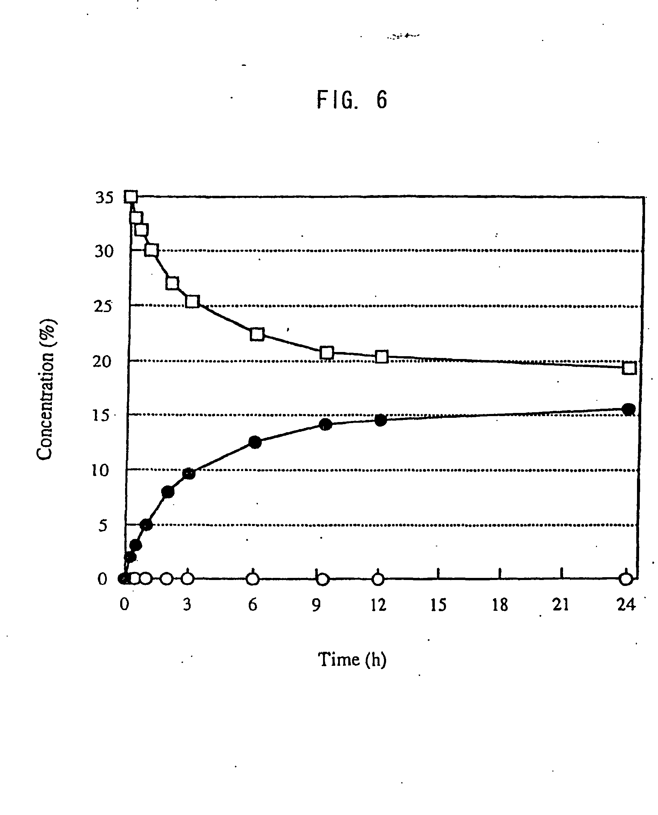

Fig. 6 shows asymmetric hydrolysis results of racemic pantolactone with immobilized enzymes. The ratio of D- and L-pantoic acid was determined by HPLC. ●: D-Pantoic acid. O: L-Pantoic acid. □: DL-Pantoyl lactone (racemic pantolactone).

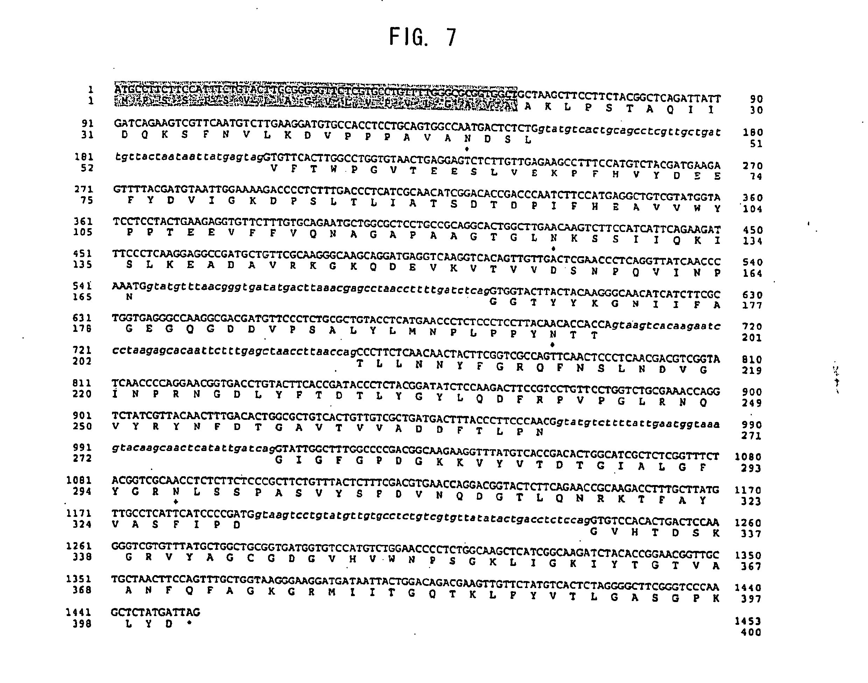

Fig. 7 shows the nucleotide sequence of chromosomal DNA (genomic gene) encoding Fusarium filamentous fungal lactonase. The NH₂-terminal signal peptide region composed of 20 amino acid residues (nucleotides 1 to 60) is indicated by shading. Five introns are shown in lowercase letter. The presumed N-glycosylated asparagine residues are indicated by diamond marks. A relevant stop codon is indicated by an asterisk.

### BEST MODES OF CARRYING OUT THE INVENTION

The present invention provides recombinant expression techniques for genes encoding lactonase enzymes with natural D-pantolactone-hydrolyzing properties, for example, Fusarium oxysporum wild type (naturally-occurring type) lactonase (D-pantolactone-hydrolyzing enzyme, or D-pantolactonase) or proteins having substantially equivalent activity thereto, and application techniques thereof. It provides preparation and cultivation/proliferation of transformed host cells with DNA comprising a nucleotide sequence encoding said lactonase; production of said proteins by utilizing said transformed host cells; and further applications of such proteins and transformed host cells. Further, the present invention provides a variety of useful means utilizing genes encoding the aforementioned lactonase, and efficient and more excellently productive production systems for D-pantolactone under applications of such designed expression vectors.

The present invention relates to lactonase characterized in that it asymmetrically hydrolyzes, among D,L-pantolactone substrates, selectively D-form; to techniques utilizing findings that, when hosts are transformed with the full-length form lactonase-coding gene comprising an NH₂-terminal signal peptide region, not only the resulting transformants will express and produce recombinant enzymes with molecular masses approximately equal to that of the wild type enzyme, but also the resultant enzymatic activity and enzyme stability will be satisfactory; and to techniques for acquisition of transformed cells having higher catalytic properties and more stable enzymatic activities, and applications thereof.

The present invention provides microbial expression and secretory production techniques for lactonases with D-pantolactone-hydrolyzing enzyme activity. Representatively, when the full-length form lactonase-coding gene is transferred into filamentous fungi, for example, the genera Aspergillus and Acremonium and others, in a form of including an N-terminal signal peptide region, the resulting transformants produce recombinant enzymes glycosylated with saccharide chains having molecular weights corresponding to that of the wild type enzyme, said enzymes are superior in enzyme stability to non-glycosylated enzymes in connection with the hydrolysis of γ-lactones such as DL-pantolactone, and further said transformant A. oryzae fungal strains have improved percent hydrolysis as compared to Fusarium oxysporum. Further, Ac. chrysogenum cases are excellent, including cases where it becomes possible to secrete lactonase extracellularly in large amounts, thereby enabling the practicable hydrolysis of DL-pantolactone at various enzyme levels (crude enzymes, purified enzymes or immobilized enzymes).

Gene recombination techniques (including recombinant DNA techniques) as can be utilized herein may be carried out by the methods described in, for example, T. Maniatis et al.,"Molecular Cloning", 2nd Ed., Cold Spring Harbor Laboratory, Cold Spring Harbor, N. T. (1989); The Japanese Biochemical Society (JBS) ed., "Zoku-Seikagaku Jikken Koza 1, Idenshi Kenkyu-Hou II", Tokyo Kagaku Dozin Co. Ltd., Japan, (1986); JBS ed., "Shin-Seikagaku Jikken Koza 2, Kakusan III (Recombinant DNA technique)", Tokyo Kagaku Dozin Co. Ltd., Japan, (1992); R. Wu ed., "Methods in Enzymology", Vol. 68, Academic Press, New York (1980) ;R. Wu et al. ed., "Methods in Enzymology", Vol. 100 & 101, Academic Press, New York (1983); R. Wu et al. ed., "Methods in Enzymology", Vol. 153, 154 & 155, Academic Press, New York (1987) etc., or by methods described in the references quoted therein or methods substantially equivalent thereto or modified methods thereof, the disclosures of which are incorporated herein by reference (hereinafter, all such techniques or methods shall be referred to as "gene recombination techniques"). These techniques can be those suited for objects and targets of the present invention wherein unique modifications and improvements are incorporated into known means.

The term "polymerase chain reaction" or "PCR" used herein usually refers to techniques described in U.S. Pat. No. 4,683,195 and other documents. For example, the PCR is an in vitro method for the enzymatic amplification of desired specific nucleotide sequences. In general, the PCR includes repetitive series of cycles wherein a primer elongation synthesis is constructed using two oligonucleotide primers capable of preferentially hybridizing with a template nucleic acid. Typically, the primers used in PCR may include those which are complementary to the internal nucleotide sequence of interest in the template. For example, preferable primer pairs as used herein may be those which are complementary to both ends of said nucleotide sequence to be amplified, or flanking regions adjacent to said nucleotide sequence. It is preferable to select a 5'-terminal primer such that at least an initiation codon is contained or the amplification can be performed including the initiation codon, and to select a 3'-terminal primer such that at least a stop codon is contained or the amplification can be performed including the stop codon. The primers include oligonucleotides made up of preferably 5 or more nucleotide bases, more preferably 10 or more nucleotide bases, and still preferably 18 to 25 nucleotide bases.

The PCR reactions can be carried out by methods known in the art or methods substantially equivalent thereto and modified methods thereof. For example, the PCR can be performed according to methods described in R. Saiki, et al., Science, 230: 1350, 1985; R. Saiki, et al., Science, 239: 487, 1988 ; H. A. Erlich ed., PCR Technology, Stockton Press, 1989 ; D. M. Glover et al. ed., "DNA Cloning", 2nd ed., Vol. 1, (The Practical Approach Series), IRL Press, Oxford University Press (1995) ; M. A. Innis et al. ed., "PCR Protocols: a guide to methods and applications", Academic Press, New York (1990)); M. J. McPherson, P. Quirke and G. R. Taylor (Ed.), PCR: a practical approach, IRL Press, Oxford (1991); M. A. Frohman et al., Proc. Natl. Acad. Sci. USA, 85, 8998-9002 (1988), and modified methods or variants thereof. The PCR methods can also be performed using commercially available kits suitable therefor, and can also be carried out according to protocols disclosed by manufacturers or distributors of the kits.

The term "oligonucleotide (s)" used herein refers to a relatively short single-stranded polynucleotide or double-stranded polynucleotides, or preferably polydeoxynucleotide(s). They can be chemically synthesized by known methods as described in Angew. Chem. Int. Ed. Engl., Vol. 28, pp.716-734 (1989), including phosphotriester, phosphodiester, phosphite, phosphoamidite, phosphonate methods, and the like. It has been typically known that the synthesis can be conveniently carried out on modified solid supports. For example, the synthesis can be carried out using an automated synthesizer and such a synthesizer is commercially available. The oligonucleotide may contain one or more modified nucleotide bases and, for example, it may contain a nucleotide base which does not naturally occur, such as inosine, or a tritylated nucleotide base. In some cases, they may contain one or more nucleotide bases tagged with a marker.

Identification of the target nucleic acids (polynucleotides) can be conducted by adaptations of hybridization techniques. The hybridization may be carried out according to methods as disclosed in documents mentioned in the aforementioned "gene recombination techniques", or substantially equivalent methods and modifications thereof. For instance, the hybridization is achieved by transferring a sample containing a nucleic acid such as DNA onto carriers including membranes such as nylon filters, as required, optionally followed by denaturation, fixation, washing, etc., and then reacting the transfers on the carrier (e.g., membrane), with labeled DNA probe fragments which are, as required, optionally denatured in a hybridization buffer.

The hybridization operations can be ordinarily conducted at about 35 to 80°C, more preferably about 50 to 65°C, for about 15 min to 36 hours, more preferably about 1 to 24 hours, but optimal hybridization conditions may be suitably selected. For example, the hybridization is carried out at about 55°C for about 18 hours. The hybridization buffers can be selected from those customarily used in the art. Examples of the hybridization buffers are Rapid hybridization buffer (Amersham), etc. The denaturation of carriers (e.g., membranes) with transfers includes techniques using an alkali denaturing solution. It is preferable to treat the carrier with a neutralizing solution and a buffer solution after the denaturation. The carrier fixation ((e.g., membrane fixation) is usually achieved by baking at about 40°to 100°C, more preferably about 70° to 90°C, for about 15 min to 24 hours, more preferably about 1 to 4 hours, but desired fixation conditions may be suitably selected. For example, the fixation is carried out by baking at about 80°C for about 2 hours. The washing of carriers (e.g., membranes) with transfers can be performed with washing solutions customarily used in the art, such as 50mM Tris-HCl buffer, pH8.0, containing 1M NaCl, 1mM EDTA and 0.1% sodium dodecyl sulfate (SDS). The carriers including membranes such as nylon filters can be selected from those customarily used in the art.

The alkaline denaturing solution, neutralizing solution and buffer solution can be selected from those conventionally used in the art. The alkaline denaturing solution may include, for example, solutions containing 0.5M NaOH and 1.5M NaCl, etc. The neutralizing solution may include, for example, 0.5M Tris-HC1 buffers (pH8.0) containing 1.5M NaCl, etc. The buffer solution may include, for example, 2X SSPE (0.36M NaCl, 20mM NaH₂PO₄ and 2mM EDTA), etc. As required, prior to hybridization, it is desired to optionally prehybridize carriers (e.g., membranes) containing transferred DNA, etc., to prevent non-specific hybridization. For the prehybridization, the sample is dipped, for example, in a prehybridization solution (50% formamide, 5 × Denhardt's solution (0.2% bovine serum albumin and 0.2% polyvinylpyrrolidone), 5xSSPH, 0.1% SDS, and 100 µg/ml thermally denatured salmon sperm DNA), etc., and reacted at about 35 to 50°C, preferably about 42°C, for about 4 to 24 hours, preferably about 6 to 8 hours. These conditions can be determined by those of skill in the art with suitably repeated experiments and preferred conditions would be selected. Labeled probe DNA fragments used in hybridization can be denatured, for example, under heating conditions at about 70 to 100°C, preferably about 100°C, for about 1 to 60 minutes, preferably about 5 minutes, etc. The hybridization is carried out by well known techniques per se in the art or according to methods analogous thereto. As used herein, the stringent conditions refer to, for example, those equivalent to hybridization in about 15 to 50 mM, preferably about 19 to 40 mM, and more preferably about 19 to 20 mM, with regard to Na ion concentration, at about 35 to 85°C, preferably about 50 to 70°C, and more preferably about 60 to 65°C with regard to temperature.

After the hybridization step is completed, the carriers (such as filters) are first washed extensively to remove labeled probes other than the labeled probe DNA fragments which specifically hybridize, and then subjected to detection steps. The filter washing process may be performed by a method suitably selected from techniques used in the art. For example, the washing is carried out in 0.5X SSC solution (SSC = 0.15M NaCl, 15mM citric acid) containing 0.1% SDS.

The hybridized nucleic acids can be detected representatively by autoradiography, but the detection may be performed by a method suitably selected from techniques used in the art. The nucleic acid bands corresponding to the detected signal are suspended in a suitable buffer solution such as SM solution (50mM Tris-HCl buffer, pH7.5, containing 100mM NaCl and 10mM MgSO₄). After the nucleic acid suspension is diluted to a suitable level, target nucleic acids can be isolated and purified. Further, the nucleic acids can be subjected to amplification. Screening treatments can be repeated plural times with hybridization techniques for target nucleic acids from nucleic acid samples including gene libraries, cDNA libraries, and others.

The lactonase as utilized herein may be isolated from the genera Fusarium, Cylindrocarpon, Gibberella, Aspergillus, Penicillium, Rhizopus, Volutella, Gliocladium, Eurotium, Nectria, Schizophyllum, Myrothecium, Neurospora, Acremonium, Tuberculina, Absidia, Sporothrix, Verticillium, and Arthroderma.

Representative examples of such microorganisms are Fusarium oxysporum IFO 5942, Fusarium semitectum IFO 30200, Cylindrocarpon tonkinense IFO 30561, Gibberella fujikuroi IFO 6349, Aspergillus oryzae ATCC 91002, Aspergillus oryzae IFO 5240, Aspergillus awamori IFO 4033, Penicillium chrysogenum IFO 4626, Rhizopus oryzae IFO 4706, Volutella buxi IFO 6003, Gliocladium catenulatum IFO 6121, Eurotium chevalieri IFO 4334, Nectria elegans IFO 7187, Schizophyllum commune IFO 4928, Myrothecium roridum IFO 9531, Neurospora crassa IFO 6067, Acremonium fusidioides IFO6813, Tuberculina persicina IFO 6464, Absidia lichtheimi IFO 4009, Sporothrix schenckii IFO 5983, Verticillium malthousei IFO 6624, Arthroderma uncinatum IFO 7865, etc.

The lactonase-coding gene as used herein can be obtained with gene recombination techniques according to WO, A, 97/10341; and Proc. Natl. Acad. Sci. USA, 95, 12787-92, 1998. For instance, cultured Fusarium oxysporum cells are disrupted and subjected to centrifugation to separate chromosomal DNA, followed by decomposition and removal of RNA. After removal operations of proteins, DNA components are purified. These operations can be conducted according to the reference document: "Shokubutsu Biotechnology Jikken Manual: Nouson Bunka Sha, p.252" (Plant Biotechnology Experiment Manual: Nouson Bunka Sha, p.252). Similarly, total RNA samples are_ extracted by AGPC (acid guanidinium thiocyanate-phenol chloroform) RNA extraction method from disrupted microbial cells, and subjected to suitable purification methods (e.g., oligo-dT cellulose column) to isolate purified mRNA products. Next, the resultant mRNA may be used as a template for cDNA synthesis with reverse transcriptase, etc. It is also possible to obtain the target DNA by PCR, or Reverse Transcription-PCR (RT-PCR; polymerase chain reaction coupled reverse transcription), using already-constructed gene libraries and suitable primers. It is possible to utilize the already-isolated lactonase gene as a probe for acquisition of the target DNA. The probe and the like may be labeled by a radioactive isotope using a commercially available labeling kit, such as the Random Prime DNA Labeling Kit (Boehringer Mannheim), etc. For example, a random-priming kit (Pharmacia LKB, Uppsala) and the like may be used to label the probe DNA with [α-³²P]dCTP (Amersham) and the like, and thus provide a probe with radioactivity.

Phage particles, recombinant plasmids, recombinant vectors and others, containing the target nucleic acids, can be isolated and purified by customary techniques used in the art. For instance, they are obtained by glycerol gradient ultracentrifugation (Molecular Cloning, a laboratory manual, ed. T. Maniatis, Cold Spring Harbor Laboratory, 2nd ed. 78, 1989), electrophoresis and other techniques. DNA can be isolated and purified from phage particles and the like by customary techniques used in the art. For instance, the resulting phages are suspended in TM solution (50mM Tris-HCl buffer, pH7.8, containing 10mM MgSO₄), etc., and treated with DNase I, RNase A, and others followed by addition of a Proteinase K mixture solution (20mM EDTA, 50 ug/ml Proteinase K and 0.5% SDS). The resultant mixture is incubated at about 65°C for 1 hr, subjected to phenol extraction and then to diethyl ether extraction, followed by precipitation with ethanol to form DNA precipitates. Next, the resultant DNA is washed with 70% ethanol, dried and dissolved in TE solution (10mM Tris-HCl buffer, pH8.0, containing 10mM EDTA). In addition, a large amount of target DNA can be obtained by subcloning, etc. For example, the subcloning can be performed with plasmid vectors, etc. in host E. coli, etc. The DNA thus subcloned can also be isolated and purified by techniques including centrifugation, phenol extraction, ethanol precipitation, etc. in the same manner as aforementioned. Sequencing of nucleotide sequences may be carried out by a dideoxy technique (such as an M13 dideoxy method), a Maxam-Gilbert method, etc. or may be carried out using a commercially available sequencing kit such as a Taq dyeprimer cycle sequencing kit or an automated nucleotide sequencer such as a fluorescent DNA sequencer.

The nucleic acids (or polynucleotides) as used herein are single- and double-stranded DNA, RNA, DNA:RNA hybrids, synthetic DNA, and others. They may be any of genome DNA (chromosomal gene DNA), genomic DNA libraries, cDNA, and synthetic DNA. The nucleotide sequences can be modified (by addition, deletion, substitution, etc.), and those thus modified may be encompassed herein. Further, the nucleic acids may include those encoding any of peptides and fragments thereof as disclosed herein, and are preferably DNA. The nucleic acids may also be chemically synthesized. In such cases, fragments may be chemically synthesized and coupled together with enzymes.

The resultant nucleic acids (including DNA) such as PCR products are typically herein subjected to electrophoresis on 1 to 2% agarose gels. Specific bands are cut out from the gel, and DNA is extracted with a commercially available kit, e.g., gene clean kit (Bio 101) and the like. The extracted DNA is cleaved with appropriate restriction enzymes and purified if necessary. Further, the 5'-end is, if necessary, phosphorylated with T4 polynucleotide kinase, etc. Subsequently the DNA is ligated into an appropriate plasmid vector including a pUC vector such as pUC18, and transfected into suitable competent cells. The cloned PCR products are sequenced and analyzed. Commercially available plasmid vectors such as p-Direct (Clontech), pCR-Script^{™} SK(+) (Stratagene), pGEM-T (Promega), and pAmp^{™} (Gibco-BRL) are useful for cloning of the PCR products. Transformation of host cells can be carried out by methods known in the art such as the calcium method, the rubidium/calcium method, the calcium/manganese method, the TFB high efficiency method, the FSB frozen competent cell method, the rapid colony method, electroporation and methods substantially equivalent thereto (D. Hanahan, J. Mol. Biol., 166: 557, 1983, etc.).

In such plasmid sequences, it is possible, for example, to contain modified codons suitable for expressing the cloned DNA in selected host cells or to construct restriction enzyme sites. It is also possible to contain control sequences, enhancer sequences, and other sequences for facilitating the expression of the target gene; linkers, adaptors and others, useful for ligating the target gene; effective sequences useful in controlling resistance to antibiotics or in controlling metabolism or in selection; and the like.

The plasmids for E. coli hosts include, for example, pBR322, pUC18, pUC19, pUC118, pUC119, pSP64, pSP65, pTZ-18R/-18U, pTZ-19R/-19U, pGEM-3, pGEM-4, pGEM-3Z, pGEM-4Z, pGEM-5Zf(-), pBluescript KS™ (Stratagene), and others. The plasmid vectors suitable for the expression in E. coli also include pAS, pKK223 (Pharmacia), pMC1403, pMC931, pKC30, and the like.

The host cells which are E. coli include those derived from the E. coli K12 strain and, for example, NM533, XL1-Blue, C600, DH1, HB101, JM109, and the like. In the gene engineering techniques of the present invention, it is possible to use restriction enzymes, reverse transcriptases known and widely used in the art, DNA-modifying enzymes, DNase, DNA polymerases, terminal nucleotidyltransferases, DNA ligases and the like to modify or convert DNA into a structure suitable for cloning the DNA fragments.

In accordance with the present invention, the lactonase may be expressed as fusion polypeptides (fusion proteins or hybrid proteins), and may be in vivo or in vitro converted or processed into those having substantially equivalent biological activity as compared to those (wild type enzymes) which naturally occur. The fusion protein expression systems usually used in gene engineering can be applied. Such fusion proteins can be purified by an affinity chromatography and the like, taking advantage of their fusion moieties. Modifications and alterations of protein structures can be performed with conventional and known techniques in the art. Examples of such techniques include the site-directed mutagenesis (site specific mutagenesis) utilizing synthetic oligonucleotides or others, PCR mutagenesis, and other methods.

In the present invention, DNA coding for not only said lactonase but also a signal peptide is constructed and utilized to produce a transformant which expresses said lactonase. Suitably utilizable examples of said lactonase-coding DNA are those derived from the genus Fusarium filamentous fungi. The useful nucleotide sequences used herein for expression of recombinant lactonase include, for example, those comprising a DNA sequence coding for full-length form lactonase and a DNA sequence coding for an NH₂-terminal signal peptide region of said lactonase. Said nucleotide sequences for expression of recombinant lactonase are also sequences comprising (a) a cDNA sequence coding for full-length form the genus Fusarium filamentous fungal lactonase or a full-length form the genus Fusarium filamentous fungal lactonase chromosomal gene DNA sequence and (b) a DNA sequence coding for an NH₂-terminal signal peptide region of said lactonase.

In preferable embodiments, sequences comprising a sequence coding for said Fusarium filamentous fungal lactonase and a sequence coding for the signal peptide region can be integrated into suitable expression vectors. Said expression vectors include those comprising one or plural filamentous fungal enhancer nucleotide sequences; those comprising a promoter region involved in the expression of the genus Fusarium filamentous fungal alkaline protease (Alp); those comprising an insert of one or plural filamentous fungal enhancer nucleotide sequences into promoter regions operable in filamentous fungi; those capable of composing the signal peptide region from a signal sequence region involved in the secretion of said Alp or a signal sequence region of said lactonase; and others.

Expression vectors suited for expression in filamentous fungi include sequences containing both the enhancer sequence on the Aspergillus oryzae α-glucosidase gene (agdA) promoter and the promoter region. Any promoter operable in filamentous fungi (mold) can be used as a promoter into which said enhancer sequence is incorporated. Representative examples of such promoters include but are not limited to, promoters of the genes coding for hydrolyzing enzymes such as α-amylase, glucoamylase, α-glucosidase, protease, lipase, cellulase, cellobiohydrase, and acetamidase; and promoters of the glycolytic enzymes genes, such as 3-phosphoglycerate kinase, glyceraldehyde-3-phosphate dehydrogenase, alcohol dehydrogenase, and enolase genes in the glycolytic pathway.

The preferable promoter includes those isolated from Aspergillus α-amylase, glucoamylase, and α-glucosidase genes, more preferably promoters for the Aspergillus oryzae α-glucosidase gene. The promoter, which is even a partial sequence, is encompassed as long as it serves as a promoter in filamentous fungi. Further, the suitable expression plasmids as used herein are those comprising the aforementioned improved promoter operable in filamentous fungi, in combination with a terminator, which may have one or more marker genes suited for selection of transformed hosts, as well as one or more replicable DNA regions in E. coli. Sites for integrating said enhancer sequence into the promoter are free of limitations as long as they are located in the promoter regions. As far as the terminator as used herein is operable in filamentous fungi, it is not particularly limited to, but includes, for example, suitably terminators for Aspergillus oryzae α-glucosidase gene, or terminators comprising a partial sequence thereof. The preferable selectable marker includes those genes selected from the group consisting of nitrate reductase (niaD), ornithine carbamoyltransferase (argB), tryptophan synthetase (trpC), acetamidase (amdS). The more preferable selectable marker gene is a nitrate reductase (niaD) gene. In these plasmids, restriction sites which are located between the promoter and the terminator are utilized to insert a DNA fragment which encodes the desired protein to be expressed according to the present invention.

The preferable expression vector for use herein includes those as disclosed in JP, A, 09-9968 (1997), JP, A, 5-268972 (1993), and other documents, as well as derivatives therefrom by known techniques. Suitable examples of such applicable vectors are plasmids pNLH2, pNAN8142, etc. When genes are ligated, synthetic DNA can be used as adaptor DNA between the genes. Said synthetic DNA may be any as long as both genes are linked in agreement with the translation reading frame and free of losing the activity of the gene of interest. For instance, expression of the desired gene with utilization of the APase gene promoter in combination with the translation initiation site and/or secretory signal can be done by constructing a fused gene containing a site, derived from the APase gene, linked in translation reading frame with the segment of the gene of interest. The APase secretory signal is linked at the downstream of the translation initiation codon in agreement with the translation reading frame, thereby allowing the extracellularly secretory production of desired materials. As far as the promoter is free of losing its function, it is even convenient to incorporate a lack of part of DNA fragments thereinto. It is possible to conveniently use even modified DNA sequences wherein nucleotide sequences have been altered at regions comprising promoters and translation initiation sites for altering functions owned by the promoter and translation initiation site, e.g., for enhancing the expression power. Further, it is also possible to use modified DNA sequences wherein nucleotide sequences have been altered at regions irresponsible for the functions of promoters and translation initiation sites.

The host into which the desired gene constructed as aforementioned is incorporated may be any organism as long as said gene is operable and expressible in such hosts. Examples of representative hosts are appropriately selected from eukaryotes such as yeast, filamentous fungi (mold), and plant. Examples of such hosts are the following microorganisms: the genera Fusarium, Cylindrocarpon, Gibberella, Aspergillus, Penicillium, Rhizopus, Volutella, Gliocladium, Eurotium, Nectria, Schizophyllum, Myrothecium, Neurospora, Acremonium, Tuberculina, Absidia, Sporothrix, Verticillium, and Arthroderma. Representative examples of such microorganisms are Fusarium oxysporum IFO 5942, Fusarium semitectum IFO 30200, Cylindrocarpon tonkinense IFO 30561, Gibberella fujikuroi IFO 6349, Aspergillus oryzae ATCC 91002, Aspergillus oryzae IFO 5240, Aspergillus awamori IFO 4033, Penicillium chrysogenum IFO 4626, Rhizopus oryzae IFO 4706, Volutella buxi IFO 6003, Gliocladium catenulatum IFO 6121, Eurotium chevalieri IFO 4334, Nectria elegans IFO 7187, Schizophyllum commune IFO 4928, Myrothecium roridum IFO 9531, Neurospora crassa IFO 6067, Acremonium fusidioides IFO6813, Tuberculina persicina IFO 6464, Absidia lichtheimi IFO 4009, Sporothrix schenckii IFO 5983, Verticillium malthousei IFO 6624, Arthroderma uncinatum IFO 7865, etc.

The host for the gene transfer with vectors carrying not only the APase gene promoter but also the desired lactonase gene ligated at the downstream of the region coding for the translation initiation site and/or secretion signal may be any organism as far as said gene is operable and expressible in such hosts. Preferably, the host can be appropriately selected from eukaryotes such as yeast, mold (filamentous fungus), and the like. In embodiments, examples of yeast include the genera Saccharomyces, Schizosaccharomyces, Pichia and the like. More specifically, the yeast includes Saccharomyces cerevisiae, Schizosaccharomyces pombe, Pichia pastoris, etc. The filamentous fungi (molds) include the genera Acremonium, Aspergillus, Fusarium Penicillium, Mucor, Neurospora, Trichoderma and the like. More specifically, examples of the mold includes Acremonium chrysogenum, Aspergillus niger, Aspergillus oryzae, Aspergillus awamori, Fusarium oxysporum, Fusarium semitectum, Penicillium chrysogenum, Mucor javanicus, Neurospora crassa, Trichoderma viride, etc. Among them, the most preferable mold is Acremonium chrysogenum. More specific examples of the host include Saccharomyces cerevisiae AH22R-, Acremonium chrysogenum ATCC11550, ATCC14553, Aspergillus oryzae IFO5240, Aspergillus awamori IFO4033, Fusarium oxysporum IFO5942, Fusarium semitectum IFO30200, Mucor javanicus IFO4570, Trichoderma viride IFO31137, and others. Among them, the most preferable host is Acremonium chrysogenum ATCC11550, etc.

Transformation for gene transfers into these hosts may include techniques in which protoplast cells prepared with suitable cell wall lytic enzymes are contacted with DNA in the presence of calcium chloride, polyethylene glycol, etc.; electroporation (see: for example, E. Neumann et al., "EMBO J", Vol. 1, pp.841 (1982), etc.); microinjection; gene gun transfer for incorporating a gene with a gun; etc.

For isolating the transformed cell efficiently, the fused gene may be inserted into a plasmid containing an appropriate selectable marker gene which is operable in the host and then the host may be transformed with the resultant plasmid. The selectable marker gene as can be used herein is any as long as the transformed cell can selectively be isolated. Representatives of such markers include hygromycin B resistant gene, and others. In general, the hosts used are required to be strains lacking functional genes for chosen selectable markers.

When the resultant transformed cells according to the present invention are cultivated, various culture conditions can vary depending on kinds of cells such as microbial strains. In general, the medium is selected from those containing assimilable sources of carbon, and nitrogen elements, which the transformant cells can assimilate or utilize as their sources. The carbon source is any as long as it is assimilable or utilizable as a source by the transformant cell. Examples thereof include saccharides (e.g., glucose, sucrose, starch, soluble starch, dextrin, etc.), paraffins and the like, as well as organic acids (e.g., acetic acid, citric acid, butyric acid, fumaric acid, benzoic acid, etc.), alcohols (e.g., methanol, ethanol, butanol, glycerin, etc.), fatty acids or esters thereof (e.g., oleic acid, stearic acid, etc.), fats and oils (soybean oil, rapeseed oil, lard, etc.), and the like. They can be used alone or in combination thereof. The nitrogen source is any as long as it is assimilable. Examples thereof include ammonium salts such as ammonium sulfate and ammonium nitrate; nitrates such as sodium nitrate and potassium nitrate; urea, peptone, casamino acids, corn steep liquor, corn gluten meal, bran, yeast extracts, dry yeast, soybean powders, cotton seed powders, meat extracts, other organic or inorganic nitrogen-containing materials and the like. They can be used alone or in the form of mixtures. It is also possible to add inorganic salts, minerals, vitamins, trace metal salts and other nutrient elements appropriately to the medium in an optional manner. The inorganic salt includes magnesium sulfate, sodium chloride, calcium carbonate, phosphates such as potassium monohydrogen phosphate and potassium dihydrogen phosphate, manganese salts, and the like. The other nutrient element includes malt extracts and the like. In addition, those customarily used in the art may be suitably selected and applied. In general, the cultivation is advantageously carried out by submerged culture under aerobic conditions. Aerobic fermentations are usually carried out for about 1 to 20 days, preferably for about 3 to 14 days, more preferably for about 3 to 10 days (culture period), at medium pH 3 to 9 at 10 to 50°C (culture temperature).

The enzyme products from said transformed cells (transformants or transfectants) acquired in the present invention can be obtained according to known methods, from various source materials, for example, enzyme-producing materials (such as transformant cells) including transformant cell cultures, disrupted cell culture mixtures, and cell extracts. For example, when the enzyme is accumulated in a culture medium, supernatants containing target materials are obtained by centrifugation or filtration. When target materials are accumulated in cells such as microbial cells, target protein material-containing supernatants are suitably obtained by various methods. For instance, microbial cells were harvested by known methods such as centrifugation and filtration after cultivation, next resuspended in a treating buffer containing a protein-denaturing agent such as guanidinium hydrochloride, stirred under cold conditions, and then subjected to centrifugation or other means to give target protein material-containing supernatants. Alternatively, microbial cells are resuspended in a buffer, and broken with glass beads, or disrupted with a French press, or using sonication or enzyme treatment, to give supernatants.

To isolate and purify target proteins from the supernatants and extracts, they can be treated by suitable combinations of widely known techniques per se for separation, isolation and purification. Such widely known techniques are, for example, salting out such as ammonium sulfate precipitation, etc.; solvent precipitation utilizing ethanol, etc.; gel filtration on Sephadex^{™}, etc.; ion exchange chromatography using carriers having, for example, an acidic group such as diethylaminoethyl or a basic group such as carboxymethyl, etc.; hydrophobic chromatography using carriers having, for example, a hydrophobic group such as butyl, octyl, or phenyl, etc.; dye (or chromophore) gel chromatography; electrophoresis; dialysis; ultrafiltration; affinity chromatography; high performance liquid chromatography; etc. When the enzymes are obtained as inclusion bodies, they may be subjected to solubilizing treatments using, for example, a denaturing agent, such as guanidine hydrochloride and urea, and, if necessary, in the presence of a reducing agent, such as 2-mercaptoethanol and dithiothreitol, whereupon active form enzymes are produced.

For enzyme materials, enzyme-producing cells per se may be used instead. Immobilized enzymes may include products prepared by immobilizing the enzyme or enzyme-producing cells according to techniques known in the art. The immobilization can be conducted by carrier bonding techniques, such as covalent binding and adsorption, crosslinking, and an encapsulation. It is also preferable to use cell immobilization techniques utilizing microbial cells entrapped in alginate gels. The immobilization can also be conducted using a coupling agent such as glutaraldehyde, hexamethylene diisocyanate and hexamethylene diisothiocyanate if necessary. Examples of the immobilization also includes monomer techniques in which monomers are gelled in polymerization, prepolymer techniques in which molecules having larger size than conventional monomers are polymerized, polymer techniques in which polymers are gelled, etc. It may include an immobilization using polyacrylamide, an immobilization using natural polymers such as alginic acid, collagen, gelatin, agar and κ-carrageenan, an immobilization using synthetic polymers such as photosetting resins and urethane polymers, and others.

The immobilizing techniques of enzymes and microbial cells and applications thereof may be carried out by the methods described in, for example, Ichiro CHIBATA (Ed.), "Koteika Kouso" (Immobilized Enzymes), p. 75, K.K. Kodansha Scientific, Tokyo, Japan (1975); Ichiro CHIBATA (Ed.), "Koteika Seitai Shokubai" (Immobilized Biological Catalysts), p. 67, K.K. Kodansha Scientific, Tokyo, Japan (1986); Tomoo SUZUKI (Supervisor), "Biseibutsu Kougaku Gijutsu Handbook (Microbial Engineering Technology Handbook)", Asakura Publishing Co., Tokyo, Japan (May, 1990); Tadayuki IMANAKA (Ed.), "Maruzen Advanced Technology <Seibutsu Kougaku Hen(Bioengineering Edition)> Biseibutsu Kogaku (Microbial Engineering)", pp. 180 to 194, Maruzen Co., Ltd. (September 30, 1993); JBS (Ed.), "Shin-Seikagaku Jikken Koza 13, Biotechnology", pp. 50 to 54, Tokyo Kagaku Dozin Co. Ltd., Japan (ISBN: 4-8079-1079-5) etc., or by methods described in the references cited therein or methods substantially equivalent thereto or modified methods thereof, the disclosures of which are incorporated herein by reference. These techniques can be those suited for objects and targets of the present invention wherein unique modifications and improvements are incorporated into known means. It may be possible to carry out the optical resolution of lactone compounds by an enzymatic asymmetric hydrolysis with the lactone hydrolyzing enzyme (such as D-pantolactone hydrolysis with cultivation of microbial cells and enzymes), as well as treatments of the resultant products in the same manner as disclosed in JP, A, 3-65,198 (1991) and JP, A, 4-144,681 (1992).

For instance, the transformed microorganisms (transformants) thus obtained are subjected to shaking culture in a liquid medium. The resulting cultured cells are harvested, to which an aqueous solution of D,L-pantolactone (concentrations: 2 to 60%) is added. The mixture is made to react at 10 to 40°C for from several hours to one day while adjusting the pH to from 6 to 8. After completion of the reaction, the cells are separated and the unreacted L-pantolactone in the reaction solution is separated by extracting with organic solvents (preferably esters such as ethyl acetate, aromatic hydrocarbons such as benzene or halogenated hydrocarbons such as chloroform). D-Pantoic acid remaining in the aqueous layer is heated under an acidic condition with hydrochloric acid to conduct a lactonation followed by extracting with the above-mentioned organic solvent whereupon the resulting D-pantolactone is obtained.

Thus, the present invention provides efficient processes for producing an optically-active γ-lactone derivative or a salt thereof, which comprise
contacting a compound of the general formula (I) or a salt thereof with a member selected from the group consisting of cell cultures, cells, and processed cell products of lactonase-producing transformants which have received a gene transfer with DNA coding for not only the genus Fusarium filamentous fungal lactonase but also a signal peptide region or a vector having an insert of said DNA, recombinant lactonase products obtained from said transformants and the like, and
separating the unhydrolyzed S-form compound (II) or a salt thereof from the reaction system, and/or
lactonizing the resultant hydrolyzed compound (III) to form a compound of the general formula (IV) or a salt thereof.

In accordance with the present invention, the aforementioned resulting transformants, particularly transformed microorganisms, are suitably used for R-selective asymmetric hydrolysis of a compound of the general formula (I), and applied to industrial production techniques for the compounds (II), and the compounds (IV) via the formation of the compounds (III). For instance, the production scheme is as follows: 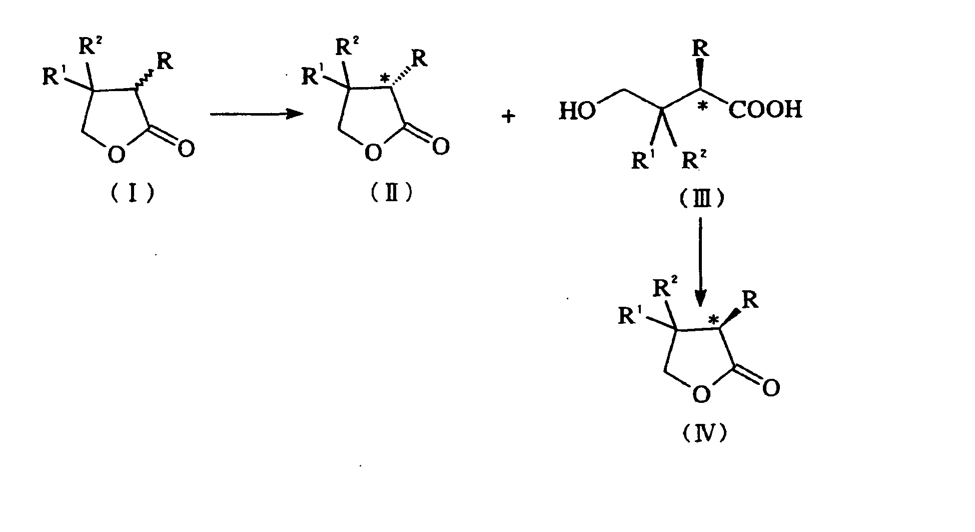 wherein * is asymmetric carbon (or optically-active carbon), R is hydroxyl or amino, R¹ and R², identical or different each other, are independently hydrogen or lower alkyl.

For R¹ and R², said lower alkyl is straight chain or branched alkyl containing 1 to 5 carbon atoms, such as methyl, ethyl, propyl, isopropyl, butyl, and t-butyl. Typically, R is hydroxyl, R¹ and R² are both methyl. In such cases, the compound (I), i.e., racemic pantolactone or a salt thereof gives an optically-active pantolactone or a salt thereof, for example, the compound (IV), i.e., D-pantolactone or a salt thereof, or the compound (II), i.e., L-pantolactone or a salt thereof.

In the processes, the applicable transformants, particularly transformed microorganisms, may include any form among cultures obtained by cultivation of microbial strains in liquid medium, microbial cells separated from culture liquid, dry microbial cells produced by processing of cells or cultures, immobilized microbial cells, and others. Further, the applicable enzymes isolated from said transformants may be any form among crude and purified products, immobilized products and others.
Operations can be done in any fashion, for example in a batch, semi-batch, or continuous fashion. The concentration of γ-lactone used is ordinarily 10 to 500 g/L. The reaction temperature is usually 10 to 50°C, more preferably 20 to 30°C. The reaction time is usually ranging from several hours to 1 day period at batch operations. The pH of such reaction systems is usually about 3 to 9, more preferably 6 to 8.

The transformant microorganisms asymmetrically hydrolyzed the compound (I) in a selective manner. As a result, the compound (III) is produced, accompanied with a decrease of the reaction solution pH, as well as with reduction in reaction rate (velocity). For accelerating the reaction, it is preferable that the reaction solution pH maintains optimal for each microbial lactone-hydrolyzing enzyme. In such cases, the inorganic salts used for maintaining desired pH are alkali metal or alkali earth metal hydroxides or carbonates, as well as aqueous ammonia solution. After completion of the reaction, the compound (II) which is nonhydrolyzed is separated from the reaction mixture by operations including extraction with an organic solvent. The organic solvent includes halogenated hydrocarbons such as ethylene chloride, chloroform, and trichloroethane; aromatic hydrocarbons such as benzene, and toluene; ethers such as diethyl ether, t-butyl methyl ether; as well as ethyl acetate and others. The preferable solvent is ethyl acetate.

Besides the extraction, the separation technique may include column chromatography and other means. It is also possible to separate the compound (II) from the compound (III) on a reverse column with an eluting solution including water, alcohols such as methanol and ethanol, and mixtures thereof.

The compound (III) remaining in the reaction liquid can be converted to the compound (IV) by acidifying the reaction solution without any treatment. The acids as used herein are hydrochloric acid, sulfuric acid, and the like, more preferably sulfuric acid. Conditions for pH, and reaction temperatures & periods may be any as long as the compound (III) can be cyclized to form the compound (IV). More preferably, the pH is acidic (pH 1 or higher), the reaction temperature is ranging from 80 to 130°C, and the reaction time from 1 to 6 hours.

The resulting compounds (IV) may be recovered by extraction with organic solvents. The solvent as used herein includes, similarly to the aforementioned solvents utilized in extraction of the compounds (II), halogenated hydrocarbons such as ethylene chloride, chloroform, and trichloroethane; aromatic hydrocarbons such as benzene, and toluene; ethers such as diethyl ether, t-butyl methyl ether; as well as ethyl acetate and others. The preferable solvent is ethyl acetate. It is also possible to carry out recovery by column chromatography.

The residual compounds (III) remaining in the reaction solution can be isolated as alkali metal carboxylates by treating the reaction mixture with alkali hydroxides, alkali carbonates, or others, then evaporating the reaction solution under reduced pressure, and finally subjecting the resultant dry solid products to recrystallization with solvents. The applicable alkali metal hydroxides or carbonates include lithium hydroxide, sodium hydroxide, potassium hydroxide, lithium carbonate, sodium carbonate, potassium carbonate, and others. The preferable alkali is sodium hydroxide. The recrystallization solvent as used herein includes methanol, ethanol, isopropanol, chloroform, and others, preferably methanol.

In particular, the present invention allows the production of D-pantolactone with high optical purity in a high yield. In the present invention, D-pantolactone can be coupled with a Ca salt or ester of β-alanine in an alcoholic solvent to produce calcium D-pantothenate. For instance, D-pantolactone is coupled with β -alanine or its ester in an organic solvent such as an alcohol to produce D-pantothenic acid, and the resultant D-pantothenic acid is then reacted with a calcium compound such as calcium carbonate to produce calcium D-pantothenate (E. Stiller, et al., J. Am. Chem. Soc., 62, 1785 (1940)). Alternatively, a mixture of D-pantolactone and β-alanine is boiled in the presence of a secondary or tertiary amine, and the resultant reaction mixture is admixed with calcium oxide to calcium D-pantothenate (E. H. Wilson, et al., J. Am. Chem. Soc., 76, 5177 (1954)), etc. It can be also coupled with 3-aminopropanol in an alcoholic solvent to produce D-panthenol (USP No. 2,413,077; Brit. Patent No. 568,355). Further, D-pantolactone can be reacted with γ-aminopropionitrile in an organic solvent such as an alcohol to produce D-pantothenonitrile, the resultant nitrile can be then reacted with cysteamine to produce 2-(2-D-pantoamidoethyl)-2-thiazoline which can be then hydrolyzed to produce D-pantetheine, and the resultant D-pantetheine can be subjected to a condensation reaction in the presence of hydrogen peroxide to produce pantethine (M. Shimizu, et al., Chem. Pharm. Bull., 13, 180 (1965)). Utilization of D-pantolactone obtainable in the present invention allows the efficient production of D-pantothenic acid and/or a salt thereof; or D-panthenol.
Thus, the present invention provides simple, convenient, efficient methods for the production of optically-active γ-lactone derivatives which are useful as intermediates for pharmaceutical drugs and amino acid derivatives.

For terms (words), symbols and/or abbreviations used in the specification and in the drawings, they must conform with the "IUPAC-IUB Commission on Biochemical Nomenclature" or are based on the meanings of the definitions or standards which are commonly or conventionally used in the art. Representative abbreviations have meanings as follows:

| | | | |
|---|---|---|---|
| A: | Alanine (Ala) | M: | Methionine (Met) |
| C: | Cysteine (Cys) | N: | Asparagine (Asn) |
| D: | Aspartic acid (Asp) | P: | Proline (Pro) |
| E: | Glutamic acid (Glu) | Q: | Glutamine (Gln) |
| F: | Phenylalanine (Phe) | R: | Arginine (Arg) |
| G: | Glycine (Gly) | S: | Serine (Ser) |
| H: | Histidine (His) | T: | Threonine (Thr) |
| I: | Isoleucine (Ile) | V: | Valine (Val) |
| K: | Lysine (Lys) | W: | Tryptophan (Trp) |
| L: | Leucine (Leu) | Y: | Tyrosine (Tyr) |

Regarding symbols for nucleotides:

| | | | |
|---|---|---|---|
| A: | Adenine | G: | Guanine |
| C: | Cytosine | T: | Thymine |

(1) The transformant Aspergillus oryzae, designated A. oryzae/pNAN-XG, receiving a gene transfer with pNAN-XG as disclosed in Example 1 mentioned below has been deposited as from February 4, 2003 (original deposit date) with the National Institute of Advanced Industrial Science and Technology (AIST; Independent Administrative Institution (IAI)), International Patent Organism Depositary (IPOD) (the former name: National Institute of Bioscience and Human Technology (NIBH), Agency of Industrial Science and Technology, Ministry of Economy, Trade and Industry (METI)), located at AIST Tsukuba Central 6, 1-1, Higashi 1-Chome, Tsukuba-shi, Ibaraki-ken 305-8566 Japan (the former address: 1-3, Higashi 1-chome, Tsukuba-shi, Ibaraki Zip Code: 305-8566, Japan) and has been assigned the Accession Number FERM P-19199. The original deposit of the transformant A. oryzae/pNAN-XG has been transferred to one under the Budapest Treaty by a request dated Feb. 2, 2004 and is on deposit with the Accession Number FERM BP-08608 under the terms of the Budapest Treaty at IPOD.
   The transformant Acremonium chrysogenum, designated Ac. chrysogenum/pAlpS, receiving a gene transfer with pAlpS as disclosed in Example 2 mentioned below has been deposited as from February 4, 2003 (original deposit date) with IPOD and has been assigned the Accession Number FERM P-19200. The original deposit of the transformant Ac. chrysogenum/pAlpS has been transferred to one under the Budapest Treaty by a request dated Feb. 2, 2004 and is on deposit with the Accession Number FERM BP-08609 under the terms of the Budapest Treaty at IPOD.
(2) The transformant E. coli, designated E. coli JM109 (EJM-ESE-1), receiving a gene transfer with having a recombinant vector (pFLC40E) having an insert of the enzyme D-pantolactone hydrolase gene, as utilized in Example 1 mentioned below has been deposited as from Aug. 30, 1995 (original deposit date) with IPOD and has been assigned the Accession Number FERM P-15141. The original deposit of the transformant E. coli JM109 (EJM-ESE-1) has been transferred to one under the Budapest Treaty by a request dated Aug. 28, 1996 and is on deposit with the Accession Number FERM BP-5638 under the terms of the Budapest Treaty at IPOD.

Details of the present invention are described by the following examples.

Specific molecular biological operations and treatment conditions in the examples as described herein below are conducted or selected according to customary techniques disclosed in standard experimental manuals, e.g., J. Sambrook, E. F. Fritsch & T. Maniatis (ed.), "Molecular Cloning: A Laboratory Manual (2nd edition)", Cold Spring Harbor Laboratory Press, Cold Spring Harbor, New York (1989); D. M. Glover et al. (ed.), "DNA Cloning", 2nd ed., Vol. 1 to 4, (The Practical Approach Series), IRL Press, Oxford University Press (1995); H. A. Erlich (ed.), PCR Technology, Stockton Press, 1989 ; D. M. Glover et al. (ed.), "DNA Cloning", 2nd ed., Vol. 1, (The Practical Approach Series), IRL Press, Oxford University Press (1995); M. A. Innis et al. (ed.), "PCR Protocols: a guide to methods and applications", Academic Press, New York (1990)); M. J. McPherson, P. Quirke and G. R. Taylor (ed.), PCR: a practical approach, IRL Press, Oxford (1991), and others. When commercially available reagents and kits are used, protocols, drugs, etc. attached thereto are employed herein.

### Example 1

### (Expression of Fusarium oxysporum Lactonase in Aspergillus oryzae)

### (1) Construction of Plasmids

A DNA fragment, which corresponds to cDNA encoding the lactonase, was isolated from pFLC40E containing an insert of Fusarium oxysporum mature lactonase cDNA (lacking an NH₂-terminal signal peptide and introns) by digestion with EcoRI and XbaI.

After this fragment was subjected to blunting, the fragment was inserted at the PmaCI site of expression plasmid pNAN8142 (JP, A, 09-9968 (1997); Biosci. Biotechnol. Biochem., 60: 383-389, 1996, wherein said PmaCI site is located at the downstream of promoter p-No8142) to form a recombinant plasmid (designated as pNAN-PC).

A plasmid, pNAN - XC, which bearing cDNA encoding the lactonase with the signal peptide, was constructed as follows:

Total RNA was isolated from F. oxysporum cells by the acid-guanidinium thiocyanate-phenol (AGPC) method (kit name: ISOGEN, Nippon Gene, Tokyo, Japan). RT-PCR amplification (technique for amplifying DNA from RNA by PCR) was performed (with Access RT-PCR System; Promega) using the total RNA as a template to amplify a 1.3 kb DNA fragment. Two oligonucleotides, FusLacl (containing an XhoI site underlined), and FusLac2 (containing an XbaI site underlined) were used as primers.
**FusLac1 ; 5' -AACTCGAGATGCCTTCTTCCATTTCTGTA-3' [SEG ID NO: 1]**
**FusLac2 ; 5' -AATCTAGACTAATCATAGAGCTTGGGAC-3' [SEQ ID NO:2]**
PCR amplifying conditions follow instructions given by the PCR kit manual. The PCR product was purified from low-melting agarose gel, digested with XhoI and XbaI, and ligated with pNAN8142 at the same restriction site.

The genomic gene of full-length form lactonase was amplified by PCR using as a template the total DNA, which was isolated from F. oxysporum, together with the oligonucleotide primers, FusLac1 and FusLac2. The amplified DNA fragment was inserted into the XhoI/XbaI site of pNAN8142, and the resulting plasmid was designated as pNAN-XG.

### (2) Expression of the lactonase gene in A. oryzae

The aforementioned plasmids were introduced individually into Aspergillus oryzae niaD mutant (AON-2), which was derived from Aspergillus oryzae (ATCC91002) according to the Unkles SE et al. method (Mol. Gen. Genet. 218, 99-104, 1989), and the lactonase activities of each transformants were examined. The enzyme assay for lactonase activities was conducted as follows:

The reaction mixture (final volume: 2ml), comprising 50 mg (wet weight) of the microbial cells, 0.5M Tris-HCl buffer (pH7.4), and 0.61 mM (80 mg) of D-pantoyl lactone, was incubated at 30°C for 15 min with reciprocal shaking (300 rpm). After removal of the cells by centrifugation, the supernatant was analyzed for pantoyl lactone and pantoic acid by high-performance liquid chromatography (HPLC). One unit (1U) enzyme is defined as the amount of enzyme catalyzing the hydrolysis of 1 µmol D-pantoyl lactone per min.

The pNAN-XG transformants showed the highest lactonase activity (about 7.4 fold as compared to F. oxysporum). The pNAN-XC transformants exhibited similar enzyme activity to the pNAN-XG transformants, but the pNAN-PC transformants produced about quarter as much enzyme as the pNAN-XG transformants (Table 1).

**Table 1**

| Vector | Number of Transformants Examined | Lactonase Activity (mU/mg (wet wt-) Cells | |
|---|---|---|---|
| | | Mean | Maximum |
| pNAN-PC | 4 | 57.0 | 69.2 |
| pNAN-XC | 7 | 195 | 255 |
| pNAN-XG | 6 | 211 | 388 |

### (3) Comparison of Recombinant Enzymes

When each cell-free extract of pNAN-XG and pNAN-XC transformants was subjected to SDS-PAGE, an extra band of 60 kDa, identical to the molecular mass of the F. oxysporum lactonase (wild type lactonase), was detected (Fig. 1A). On the other hand, the cell-free extract of pNAN-PC transformant gave an extra band of 51 kDa on SDS-FACE. When the cell-free extracts were analyzed using Western blotting with anti-lactonase antiserum, a positive band at 51 kDa was observed for pNAN-PC transformants while a positive band at 60 kDa was for pNAN-XG transformants (Fig. 1B). Similarly, a positive band at 60 kDa was observed for pNAN-XC transformants. Each N-terminal amino acid sequence was analyzed (sequenced) for individual recombinant enzymes from pNAN-XG and pNAN-PC transformant cells. As a result, it was confirmed that both enzymes had the same N-terminal amino acid sequence (Ala-Lys-Leu-Pro-Ser) as the naturally-occurring one, and were correctly processed. Therefore, the difference between the molecular weights of individual recombinant enzymes has been considered to be due to the sugar modification of the enzymes.
Because only the pNAN-PC transformant wherein the recombinant plasmid lacks the signal peptide DNA produces deglycosylated form enzymes, it has been supposed that the signal peptide of the lactonase is essential for the enzyme polypeptide to be transferred to endoplasmic reticulum (ER) where proteins are glycosylated after the translation.

### (4) Asymmetric Hydrolysis of Racemic Pantoyl Lactone

An aqueous 35% solution of DL-pantolactone was incubated with F. oxysporum or A. oryzae transformants (pNAN-PC or pNAN-XG) while controlling the pH in the range from 6.8 to 7.5. Although pNAN-PC transformant cells hydrolyzed the substrate with high initial velocity, the stability of the enzyme was quite low and the hydrolysis reaction hardly proceeded after 2 hr. After the reaction for 24 hr, the percent hydrolysis for DL-pantoyl lactone was 8.52% (Fig. 2A), which was inferior to the result for F. oxysporum cells (14.6%). The recombinant enzyme of pNAN-XG transformants was more stable than that of pNAN-PC transformants, and had the percent hydrolysis of 19.8%.

In order to improve the enzyme stability for attaining the desired percent hydrolysis value, i.e., 50%, the wet cells of microorganisms (5 g) were thoroughly suspended in 115 ml of 1% sodium alginate, and dripped into an aqueous 2% solution of CaCl₂ to give immobilized transformant cells. In the asymmetric hydrolysis where the immobilized recombinant cells were applied, the stability of both pNAN-XG and pNAN-PC transformant-derived enzymes was improved and their hydrolysis percentage of DL-pantolactone increased remarkably. After the reaction for 24 hr, the percent hydrolysis reached 49.1 % for the immobilized pNAN-XG transformant cells. For the immobilized pNAN-PC transformant cells, the percent hydrolysis reached 21.0% (FIG. 2B). Each optical purity of products was >95% e.e.

### Example 2

### (Secretory Expression of Fusarium oxysporum Lactonase by Acremonium chrysogenum)

### (1) Construction of Plasmids

Combined PCRs were carried out to construct fusion genes comprised of the Fusarium alkaline protease (Alp) promoter, signal sequences thereof and the lactonase gene (Fig. 3).

To amplify the Alp promoter followed by genes encoding the Alp signal peptide and propeptide, PCR was performed using pNLH2 (JP, A, 5-268,972 (1993)) as a template and oligonucleotides as primers. The primers used are as follows:

**AlpPS: 5' -TTGTCGACGGATCCGAAAGATGAGACCGCT-3'** **[SEQ ID NO: 3]**

**AlpPA; 5'-GAAGCTTAGCCATGTTGATGCTGATGATGGCATCC-3'** **[SEQ ID NO: 4]**

**LacS; 5'-CATCAGCATCAACATGGCTAAGCTAAGCTTCCTTCTACGGCTC-3'** **[SEQ ID NO: 5]**

Sense primer: AlpPS
(the underlined is a synthesized SalI site)
Antisense primer: AlpPA
(the underlined is capable of annealing with part of the primer LacS)

The lactonase gene, lacking the NH₂-terminal signal peptide-coding region, was also amplified by PCR with the sense primer LacS (the underlined sequence is capable of annealing with part of the primer AlpPA) and the antisense primer FusLac2 (SEQ ID NO: 2) in combination with pNAN-XG as a template. Each mixed PCR product was subjected to a 2nd PCR amplification with primers AlpPS and FusLac2. The PCR resultant product (2.3 kb) was digested with SalI and XbaI, and then subcloned into pBluescript, giving pAlpS.

Another expression vector, pLacS, which was designed to link the Alp promoter to the lactonase gene with its own signal peptide, was also constructed by combined PCRs. Briefly, the Alp promoter was amplified by PCR with primers, AlpPS and AlpPA2 (the underlined sequence is capable of annealing with part of primer LacS2), and the gene encoding full-length form lactonase together with its own signal peptide was amplified by PCR with primers, LacS2 (the underlined sequence is capable of annealing with part of primer AlpAP2) and FusLac2.

**AlpPA2: 5'-TGGAAGAAGGCATCTTGTCAGGGAGTATGAAGGTTG-3'** **[SEQ ID NO: 6]**

**LacS2: 5' -TACTCCCTGACAAGATGCCTTCTTCCTTTCTGTA-3'** **[SEQ ID NO: 7]**

Both PCR products were mixed and subjected to 2nd PCR using AlpPS and FusLac2, and the resulting amplified product was digested by SalI and XbaI, followed by subcloning into pBluescript. A 3.0 kb DNA fragment, including the Ac. Chrysogenum glyceraldehyde-3-phosphate dehydrogenase (GAPDH) promoter and hygromycin B phosphotransferase gene, was obtained by the digestion of pNLH2 (developed by Morita, S et al.; J. Biotechenol. 42, 1-8, 1995) with HindIII, and then inserted into HindIII site of pBluescript to form pHm^{R}.

### (2) Expression of lactonase genes in Ac. Chrysogenum

Each of the aforementioned plasmids was introduced into Ac. Chrysogenum (ATCC11550). In order to confirm that the lactonase genes were inserted into the chromosomal DNAs of the transformants PCR analysis was carried out using, as primers, oligonucleotides corresponding to 5'- and 3'-terminal regions of the lactonase gene in combination with, as a template, each genomic DNA from the transformants. When the PCR products were electrophoresed on agarose gel, 1.2 kb bands corresponding to the lactonase gene were detected among 80 examined transformants at about 80% of the transformants.

Each transformant was precultured in a medium consisted of 30 g sucrose. 5 g DL-methionine, 32 g soy bean flour, and 1.5 g CaCO₃ per liter (pH 6.8) at 28°C for 3 days. Aliquots of these cultures were inoculated into a main medium (500 ml) and incubated at 28°C for 5 days with shaking (120 strokes/min). Each culture supernatant of cultivated transformants was assayed for lactonase activity.

The lactonase activity was analyzed as follows:
After a reaction mixture consisted of 0.5M Tris-HCl buffer (pH 7.4), 0.61 mM D-pantoyl lactone (80 mg), and an appropriate amount of enzymes in a final volume of 0.5 mL is incubated at 30°C for 15 min, the mixture was assayed for pantoyl lactone and pantoic acid by HPLC. As a result, large amounts of the lactonase were observed in culture supernatants of pAlpS and pLacS transformants (Table 2).

**Table 2**

| Vector | Number of Transformants Examined | Extracellular Lactonase (mg/l) | |
|---|---|---|---|
| | | Mean | Maximum |
| pAlpS | 10 | 57.9 | 190 |
| pLacS | 10 | 43.4 | 88.8 |

### (3) Characterization of Secreted Enzymes

When the culture supernatants of pAlpS and pLacS transformants were subjected to SDS-PAGE, major bands at molecular mass 60kDa were detected (Fig. 4). This molecular mass is identical to that of the wild type lactonase. The proteins corresponding to these bands were transferred onto PVDF membrane, and applied to NH₂-terminal amino acid sequencing. Both pAlpS and pLacS transformant-derived proteins have the same amino acid sequence (Ala-Lys-Leu-Pro-Ser-Thr-Ala-) as the wild type enzyme.

For structurally analyzing the saccharide chains of enzymes, purified enzyme samples were treated with endoglycosidase, which cleaves only high-mannose type and several hybrid oligosaccharides from N-linked glycoproteins. The purified enzyme samples were recombinant proteins isolated from F. oxysporum cells and from pAlpS transformant cells. Each purified enzyme sample was incubated with endoglycosidase for 1, 5, 15, or 60 min, and then subjected to SDS-PAGE. As a result, three bands of different molecular sizes (60, 56, and 51 kDa) were observed (Fig. 5). This fact indicates each enzyme carries at least two N-linked saccharide chains.

### (4) Preparation of Immobilized Enzymes

A culture supernatant (500 ml) from pAlpS transformant cells was dialyzed against 20 mM Tris-HCl buffer (pH7.4), concentrated to 100 ml by ultrafiltration with Amicon YM-10 membrane (Millipore, Bedford, MA. USA; cut off 10,000), and used as an enzyme solution.

For carriers, Deolite A-568 was washed with 0.1M NaOH, and then washed with purified water until the pH of the suspension was dropped to 8.2. To the enzyme solution (100 ml) was added 15 g (as dry wt.) of washed Deolite A-568, the resultant mixture was stirred at 4°C for 24 hr to adsorb the enzyme. After adsorption, the resins were washed with purified water, and crosslinked with 100 ml of 2% glutaraldehyde in 20 mM Tris-HCl buffer (pH 7.4) at 4°C for 24hr.

Unimmobilized proteins remained in the culture supernatant at about 13.6% of the proteins put on. The D-lactonase activity of immobilized enzymes was estimated to be 60.4 U/g wet resin, and the activity yield was 14.8% (Table 3).

**Table 3**

| Activity put on (U/g-Resin (dry wt.)) | Protein put on (mg/g-Resin (dry wt.) | Activity Adsorbed (U/g) | Activity Yield (%) | Protein Adsorbed (mg/g) | Protein Yield (%) |
|---|---|---|---|---|---|
| 858 | 9.07 | 127 | 14.8 | 7.83 | 86.4 |

### (5) Asymmetric Hydrolysis of Racemic Pantolactone with Immobilized Enzymes

A 35% (w/v) solution (75 mL) of DL-pantolactone was incubated with 15 g (wet wt.) of the enzyme-immobilized resins at 30°C with gentle stirring while the pH was controlled at 6.8 to 7.5. It was observed that the asymmetric hydrolysis proceeded efficiently and the byproduct, i.e., L-pantoic acid, was not detected (Fig. 6). After the reaction for 9 hr, the hydrolysis percentage for racemic substrates exceeded 40%, i.e., the D-form substrate was hydrolyzed at 80%. The percent hydrolysis reached 45% after 24 hours.

### INDUSTRIAL APPLICABILITY

The present invention allows the development of manufacturing techniques for efficient and excellently productive systems capable of producing the lactonase enzyme wherein said system utilizes the naturally-occurring (native) lactonase gene. The present invention also allows the construction of efficient and advantageously productive systems for producing optically-active y -lactone, with applications of said enzyme and said enzyme-producing system. It is also possible to construct systems capable of producing D-pantothenic acid, D-panthenol, pantethine and others, which are pharmaceutically and physiologically important vitamins. The present invention is useful in the production of pharmaceutical drugs, as well as food and feed additives, cosmetic products.

While the present invention has been described specifically in detail with reference to certain embodiments and examples thereof, it would be apparent that it is possible to practice it in other forms. In light of the disclosure, it will be understood that various modifications and variations are within the scope of the appended claims.

### [Sequence Listing Free Text]

SEQ ID NO: 1, Description of Artificial Sequence:
   Oligonucleotide to act as a primer for PCR
SEQ ID NO: 2, Description of Artificial Sequence:
   Oligonucleotide to act as a primer for PCR
SEQ ID NO: 3, Description of Artificial Sequence:
   Oligonucleotide to act as a primer for PCR
SEQ ID NO: 4, Description of Artificial Sequence:
   Oligonucleotide to act as a primer for PCR
SEQ ID NO: 5, Description of Artificial Sequence:
   Oligonucleotide to act as a primer for PCR
SEQ ID NO: 6, Description of Artificial Sequence:
   Oligonucleotide to act as a primer for PCR
SEQ ID NO: 7, Description of Artificial Sequence:
   Oligonucleotide to act as a primer for PCR

## Claims

1. A lactonase-producing transformant of the genus *Acremonium* or *Aspergillus* which has genetically acquired a DNA comprised of a lactonase-encoding DNA coding for amino acid residues 1 to 380 of SEQ ID NO:9 and a signal peptide region-encoding DNA coding for amino acid residues -20 to -1 of SEQ ID NO:9.

2. A process for the production of a recombinant lactonase with D-pantolactone-hydrolyzing enzyme activity, which comprises
culturing the lactonase-producing transformant of claim 1 to produce the recombinant lactonase, and
isolating the resultant recombinant lactonase from the culture.

3. A process for producing an optically-active γ-lactone derivative of the general formula (II): 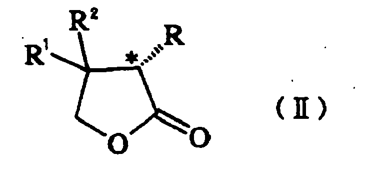 wherein R is hydroxyl or amino, R¹ and R², identical or different each other, are independently hydrogen or lower alkyl,or of the general formula (IV): 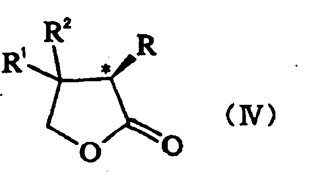 wherein R, R¹ and R², have the same meanings as defined above, or a salt thereof, which comprises contacting a compound of the general formula (I): 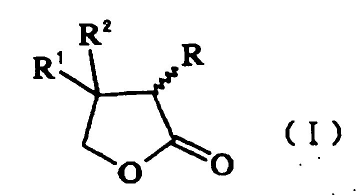 wherein R, R¹ and R², have the same meanings as defined above, or a salt thereof,
with a member selected from the group consisting of cell cultures, cells, and immobilized cells of the lactonase-producing transformant of claim 1 to produce the optically-active γ-lactone derivative (II) or (IV), or a salt thereof.

4. The process of claim 3, wherein the optically active γ-lactone of formula (IV) is D-pantolactone, or a salt thereof, and the process allows the production of D-pantolactone derivatives or salts thereof.

5. The process of claim 4, wherein the D-pantolactone derivatives are selected from pantothenic acid, calcium pantothenate, panthenol, pantetheine, panthenyl ethyl ether and pantethine.

## Patentansprüche

1. Lactonase-produzierende Transformante der Gattung *Acremonium* oder *Aspergillus,* die genetisch eine DNA erworben hat, welche aus einer Lactonase-codierenden DNA, die für die Aminosäurereste 1 bis 380 von SEQ ID Nr. 9 codiert, und einer Signalpeptidbereich-codierenden DNA, die für die Aminosäurereste -20 bis -1 von SEQ ID Nr. 9 codiert, besteht.

2. Verfahren zur Herstellung einer rekombinanten Lactonase mit D-Pantolacton-hydrolysierender Enzymaktivität, umfassend:
Kultivieren der Lactonase-produzierenden Transformante gemäß Anspruch 1 unter Bildung der rekombinanten Lactonase; und
Isolieren der resultierenden rekombinanten Lactonase aus der Kultur.

3. Verfahren zur Herstellung eines optisch aktiven γ-Lacton-Derivats der allgemeinen Formel (II) 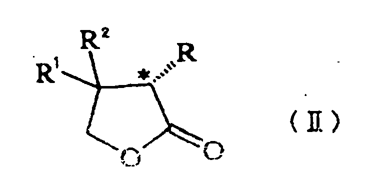 wobei R Hydroxy oder Amino ist, R¹ und R² gleich oder verschieden sind und unabhängig voneinander Wasserstoff oder Niederalkyl sind, oder der allgemeinen Formel (IV) 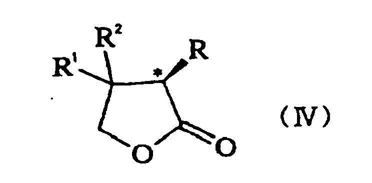 wobei R, R¹ und R² dieselben Bedeutungen haben, wie es oben definiert ist, oder eines Salzes davon, umfassend das In-Kontakt-Bringen einer Verbindung der allgemeinen Formel (I) 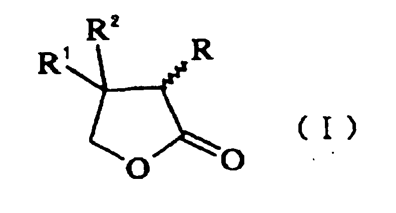 wobei R, R¹ und R² dieselben Bedeutungen haben, wie es oben definiert ist, oder eines Salzes davon mit einem Vertreter, der aus der Gruppe ausgewählt ist, die aus Zellkulturen, Zellen und immobilisierten Zellen der Lactonase-produzierenden Transformante gemäß Anspruch 1 besteht, unter Bildung des optisch aktiven γ-Lacton-Derivats (II) oder (IV) oder eines Salzes davon.

4. Verfahren gemäß Anspruch 3, wobei es sich bei dem optisch aktiven γ-Lacton der Formel (IV) um D-Pantolacton oder ein Salz davon handelt und das Verfahren die Produktion von D-Pantolacton-Derivaten oder Salzen davon ermöglicht.

5. Verfahren gemäß Anspruch 4, wobei die D-Pantolacton-Derivate aus Pantothensäure, Calciumpantothenat, Panthenol, Pantethein, Panthenylethylether und Pantethin ausgewählt sind.

## Revendications

1. Transformant producteur de lactonase du genre *Acremonium* ou *Aspergillus* qui a acquis génétiquement un ADN constitué d'un ADN codant pour la lactonase, qui code pour les résidus d'acides aminés 1 à 380 de SEQ ID NO:9 et d'un ADN codant pour la région du peptide signal, qui code pour les résidus d'acides aminés -20 à -1 de SEQ ID NO:9.

2. Procédé pour la production d'une lactonase recombinante ayant une activité enzymatique d'hydrolyse de D-pantolactone, qui comprend:
la mise en culture du transformant producteur de lactonase de la revendication 1 pour produire la lactonase recombinante, et
l'isolement de la lactonase recombinante résultante à partir de la culture.

3. Procédé de production d'un dérivé de γ-lactone optiquement actif de formule générale (II): 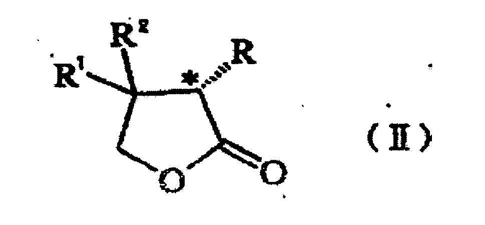 dans laquelle R est un groupe hydroxyle ou amino, R¹ et R², qui sont identiques ou différents l'un de l'autre, représentent indépendamment un atome d'hydrogène ou un groupe alkyle inférieur, ou de formule générale (IV): 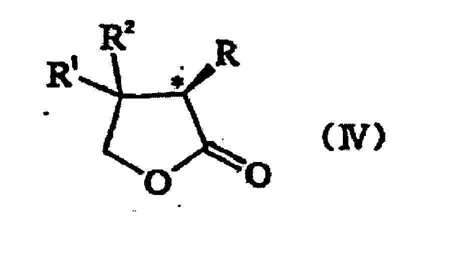 dans laquelle R, R¹ et R² ont les mêmes significations que celles définies ci-dessus, ou de l'un de ses sels, qui comprend la mise en contact d'un composé de formule générale (I): 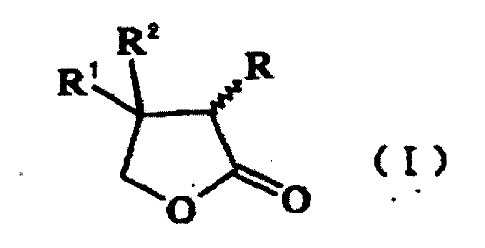 dans laquelle R, R¹ et R² ont les mêmes significations que celles définies ci-dessus, ou de l'un de ses sels,
avec un membre choisi dans le groupe constitué par les cultures cellulaires, les cellules et les cellules immobilisées du transformant producteur de lactonase de la revendication 1 pour produire le dérivé de γ-lactone optiquement actif (II) ou (IV), ou un sel d'un tel dérivé.

4. Procédé selon la revendication 3, dans lequel la γ-lactone optiquement active de formule (IV) est la D-pantolactone, ou l'un de ses sels, et le procédé permet la production de dérivés de D-pantolactone ou de leurs sels.

5. Procédé selon la revendication 4, dans lequel les dérivés de D-pantolactone sont choisis parmi l'acide pantothénique, le pantothénate de calcium, le panthénol, la pantéthéine, l'éther éthylique de panthényle et la pantéthine.
